# EUROPEAN PATENT APPLICATION

(11) **EP 4 671 280 A1**
(43) Date of publication of application: **31.12.2025**
(21) Application number: 24759707.3
(22) Date of filing: 21.02.2024
(51) Int. Cl.: C07K 16/30, C07K 14/46, C12N 15/13, C12N 15/85, A61K 39/395, A61P 35/00

(54) **ANTI-NECTIN-4 ANTIBODY AND MULTISPECIFIC ANTIBODY CONTAINING SAME**

(30) Priority: 22.02.2023 WO PCT/CN2023/077654
(71) Applicant: Nanjing Leads Biolabs Co., Ltd., Jiangbei New Area Nanjing Jiangsu 210031 (CN)
(72) Inventor: DANG, Yujia, Nanjing, Jiangsu 210019 (CN); HU, Shuijun, Nanjing, Jiangsu 210019 (CN); LI, Fengxia, Nanjing, Jiangsu 210019 (CN); HUANG, Xiao, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2024/077935
(87) International publication number: WO 2024/175030

(57) **Abstract**

The present invention relates to an isolated antibody or antigen-binding fragment specifically binding to the Nectin-4 protein, and a multispecific antibody containing the isolated antibody or antigen-binding fragment specifically binding to the Nectin-4 protein. The present invention also relates to a nucleic acid encoding the isolated antibody or antigen-binding fragment specifically binding to the Nectin-4 protein and the multispecific antibody, a host cell containing the isolated antibody or antigen-binding fragment specifically binding to the Nectin-4 protein and the multispecific antibody, and a method for preparing the isolated antibody or antigen-binding fragment specifically binding to the Nectin-4 protein and the multispecific antibody. Furthermore, the present invention relates to the diagnostic, preventive and/or therapeutic use of the isolated antibody or antigen-binding fragment specifically binding to the Nectin-4 protein and the multispecific antibody.

## Description

### TECHNICAL FIELD

The present disclosure generally relates to an antibody and use thereof. More particularly, the present disclosure relates to an isolated antibody or an antigen-binding fragment specifically binding to Nectin-4, and a multispecific antibody comprising same. The present disclosure further relates to diagnostic, prophylactic, and/or therapeutic use of the isolated antibody or the antigen-binding fragment specifically binding to Nectin-4 and the multispecific antibody.

### BACKGROUND

First-generation immune checkpoint inhibitors (ICIs) represented by PD-l monoclonal antibodies and/or PD-L1 monoclonal antibodies benefit a limited population, with only a small proportion (10% to 30%) of tumor patients showing therapeutic effects after administration of PD-1 monoclonal antibodies and/or PD-L1 monoclonal antibodies (Lawrence P. Andrews et al., (2020) Sci Immunol. 5(49): eabc2728). Consequently, over the past few years, combining drugs to increase ICI efficacy and overcome resistance to immunotherapy has become a research hotspot in academia and the pharmaceutical industry.

Nectin-4 is a 66 kDa type I transmembrane glycoprotein belonging to the Nectin family of immunoglobulin-like adhesion molecules. Members of this family mediate cell-cell adhesion, recruit cadherins, and regulate cytoskeletal rearrangements. Nectin-4 is highly expressed in bladder cancer, breast cancer, lung cancer, pancreatic cancer, ovarian cancer, etc., while its expression level is very low in normal tissues, making it an ideal tumor-associated antigen target (Pia M. Challita-Eid et al., (2016) Cancer Res. 76(10): 3003-13).

In 2020, it was reported in J Immunother Cancer that Nectin-4 is a ligand for the immune checkpoint TIGIT, and Nectin-4 antibodies block the binding of Nectin-4 to TIGIT, thereby playing a role in killing tumors by the Nectin-4 antibodies (Adi Reches et al., (2020) J Immunother Cancer. 8(1): e000266).

Nectin-4-targeted PADCEV^{™} (also known as enfortumab vedotin-ejfv) was approved by the FDA for the treatment of locally advanced or metastatic urothelial carcinoma, demonstrating that Nectin-4 is a relatively good tumor-associated antigen. PADCEV^{™} is an ADC drug targeting a Nectin-4 protein highly expressed on the cell surface in urothelial carcinoma, which is formed by conjugating the human IgG1 monoclonal antibody enfortumab targeting the Nectin-4 protein with the cytotoxic formulation MMAE (monomethyl auristatin E, a microtubule disruptor) (Pia M. Challita-Eid et al., (2016) Cancer Res. 76(10): 3003-13). However, PADCEV^{™} can cause serious adverse skin reactions, including Stevens-Johnson syndrome (SJS) and toxic epidermal necrolysis (TEN) (FDA Publication, 2019).

4-1BB, also known as CD137 or TNFRSF9, is a member of the tumor necrosis factor receptor family. Its most prominent feature is acting as a co-stimulatory molecule on the surface of T cells that regulates TCR-induced T cell activation. Upon binding of 4-1BB to 4-1BBL (the 4-1BBL is the primary natural ligand of 4-1BB expressed on activated antigen-presenting cells), anti-apoptotic molecules are up-regulated, and the production of cytokines and effector functions are promoted. 4-1BB is also present on dendritic cells, activated monocytes, NK cells, neutrophils, eosinophils, and mast cells, and 4-1BB signaling has been reported to stimulate IFN-γ secretion and promote NK cell proliferation and dendritic cell activation (Cariad Chester et al., (2018) Blood 131(1): 49-57).

Preclinical results of agonistic monoclonal antibodies against 4-1BB in a variety of induced and spontaneous tumor models suggest that targeting 4-1BB with the agonistic antibodies can result in tumor clearance and a long-lasting anti-tumor immune effect.

Although clinical trials of 2 agonistic antibodies, urelumab and utomilumab, have shown preliminary signs of efficacy, inflammatory hepatotoxicity at doses >1 mg/kg hinders the clinical development of urelumab. Utomilumab, while exhibiting relatively good safety, has a lower 4-1BB agonistic effect compared with urelumab (Cariad Chester et al., (2018) Blood 131(1): 49-57).

In order to make the best use of the immune activation ability mediated by 4-1BB, the next-generation 4-1BB targeting strategy is to improve the anti-tumor effect while reducing hepatotoxicity. Attempts have been made to limit the action of 4-1BB agonists in the tumor microenvironment to reduce the incidence of immune-related adverse events. 4-1BB is a potent target for tumor immunotherapy, showing great promise for achieving strong immune activation (Cariad Chester et al., (2018) Blood 131(1): 49-57).

Thus, there remains a need in the art to develop novel antibodies that achieve an optimal balance between safety and agonism. The present disclosure satisfies this need by providing an anti-Nectin-4 antibody binding to Nectin-4 with high target specificity and high affinity, and a bispecific antibody comprising the anti-Nectin-4 and anti-4-1BB binding to 4-1BB with low affinity.

### SUMMARY

Through research, the inventors have developed a group of novel isolated anti-Nectin-4 antibodies binding to Nectin-4 with high affinity. The isolated antibody or the antigen-binding fragment specifically binding to Nectin-4 of the present disclosure has one or more of the following properties:
(a) having an ability to bind to a human Nectin-4 molecule expressed on a cell and a cynomolgus monkey Nectin-4 molecule expressed on a cell, as measured by flow cytometry; and
(b) having an ability to specifically bind only to Nectin-4, with no binding to Nectin family members Nectin-1, Nectin-2, and Nectin-3, as measured by ELISA; and/or
(c) binding to a human Nectin-4 protein with a binding dissociation equilibrium constant K_{D} of less than about 10 nM, such as about 1 nM, about 10⁻¹ nM, about 10⁻² nM, or about 10⁻³ nM; and/or binding to a cynomolgus monkey Nectin-4 protein with a binding dissociation equilibrium constant K_{D} of less than about 100 nM, such as about 10 nM, about 1 nM, about 10⁻¹ nM, or about 10⁻² nM, as measured by biolayer interferometry.

Therefore, in a first aspect, the present disclosure provides an isolated antibody or an antigen-binding fragment specifically binding to a Nectin-4 protein, which comprises
(a) 3 CDRs in the amino acid sequence of a heavy chain variable region set forth in SEQ ID NO: 1 and 3 CDRs in the amino acid sequence of a light chain variable region set forth in SEQ ID NO: 2; or a variant with one or more CDRs each having no more than 3 amino acid modifications relative to the 6 CDR regions described above;
   for example, 3 CDRs in the amino acid sequence of a heavy chain variable region set forth in SEQ ID NO: 18 and 3 CDRs in the amino acid sequence of a light chain variable region set forth in any one of SEQ ID NOs: 20-24; or
   3 CDRs in the amino acid sequence of a heavy chain variable region set forth in SEQ ID NO: 19 and 3 CDRs in the amino acid sequence of a light chain variable region set forth in any one of SEQ ID NOs: 20-24;
(b) 3 CDRs in the amino acid sequence of a heavy chain variable region set forth in SEQ ID NO: 3 and 3 CDRs in the amino acid sequence of a light chain variable region set forth in SEQ ID NO: 4; or a variant with one or more CDRs each having no more than 3 amino acid modifications relative to the 6 CDR regions described above; or
(c) 3 CDRs in the amino acid sequence of a heavy chain variable region set forth in SEQ ID NO: 5 and 3 CDRs in the amino acid sequence of a light chain variable region set forth in SEQ ID NO: 6; or a variant with one or more CDRs each having no more than 3 amino acid modifications relative to the 6 CDR regions described above.

In some embodiments, the present disclosure provides an isolated antibody or an antigen-binding fragment specifically binding to a Nectin-4 protein, which comprises, according to Kabat numbering:
(a) an HCDR1 set forth in SEQ ID NO: 40 or a variant of the HCDR1 set forth in SEQ ID NO: 40 having no more than 2 amino acid modifications, an HCDR2 set forth in SEQ ID NO: 41 or a variant of the HCDR2 set forth in SEQ ID NO: 41 having no more than 3 amino acid modifications, and an HCDR3 set forth in SEQ ID NO: 42 or a variant of the HCDR3 set forth in SEQ ID NO: 42 having no more than 2 amino acid modifications; an LCDR1 set forth in SEQ ID NO: 43 or a variant of the LCDR1 set forth in SEQ ID NO: 43 having no more than 3 amino acid modifications, an LCDR2 set forth in SEQ ID NO: 44 or a variant of the LCDR2 set forth in SEQ ID NO: 44 having no more than 3 amino acid modifications, and an LCDR3 set forth in SEQ ID NO: 45 or a variant of the LCDR3 set forth in SEQ ID NO: 45 having no more than 2 amino acid modifications;
(b) an HCDR1 set forth in SEQ ID NO: 46 or a variant of the HCDR1 set forth in SEQ ID NO: 46 having no more than 2 amino acid modifications, an HCDR2 set forth in SEQ ID NO: 47 or a variant of the HCDR2 set forth in SEQ ID NO: 47 having no more than 3 amino acid modifications, and an HCDR3 set forth in SEQ ID NO: 48 or a variant of the HCDR3 set forth in SEQ ID NO: 48 having no more than 2 amino acid modifications; an LCDR1 set forth in SEQ ID NO: 49 or a variant of the LCDR1 set forth in SEQ ID NO: 49 having no more than 3 amino acid modifications, an LCDR2 set forth in SEQ ID NO: 50 or a variant of the LCDR2 set forth in SEQ ID NO: 50 having no more than 3 amino acid modifications, and an LCDR3 set forth in SEQ ID NO: 51 or a variant of the LCDR3 set forth in SEQ ID NO: 51 having no more than 2 amino acid modifications; or
(c) an HCDR1 set forth in SEQ ID NO: 52 or a variant of the HCDR1 set forth in SEQ ID NO: 52 having no more than 2 amino acid modifications, an HCDR2 set forth in SEQ ID NO: 53 or a variant of the HCDR2 set forth in SEQ ID NO: 53 having no more than 3 amino acid modifications, and an HCDR3 set forth in SEQ ID NO: 54 or a variant of the HCDR3 set forth in SEQ ID NO: 54 having no more than 2 amino acid modifications; an LCDR1 set forth in SEQ ID NO: 55 or a variant of the LCDR1 set forth in SEQ ID NO: 55 having no more than 3 amino acid modifications, an LCDR2 set forth in SEQ ID NO: 56 or a variant of the LCDR2 set forth in SEQ ID NO: 56 having no more than 3 amino acid modifications, and an LCDR3 set forth in SEQ ID NO: 57 or a variant of the LCDR3 set forth in SEQ ID NO: 57 having no more than 2 amino acid modifications.

Preferably, the isolated antibody or the antigen-binding fragment specifically binding to the Nectin-4 protein comprises, according to Kabat numbering:
(a) an HCDR1 set forth in SEQ ID NO: 40, an HCDR2 set forth in SEQ ID NO: 41, and an HCDR3 set forth in SEQ ID NO: 42; an LCDR1 set forth in SEQ ID NO: 43, an LCDR2 set forth in SEQ ID NO: 44, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 59, an LCDR2 set forth in SEQ ID NO: 61, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 59, an LCDR2 set forth in SEQ ID NO: 44, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 43, an LCDR2 set forth in SEQ ID NO: 62, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 60, an LCDR2 set forth in SEQ ID NO: 62, and an LCDR3 set forth in SEQ ID NO: 45;
(b) an HCDR1 set forth in SEQ ID NO: 46, an HCDR2 set forth in SEQ ID NO: 47, and an HCDR3 set forth in SEQ ID NO: 48; an LCDR1 set forth in SEQ ID NO: 49, an LCDR2 set forth in SEQ ID NO: 50, and an LCDR3 set forth in SEQ ID NO: 51; or
(c) an HCDR1 set forth in SEQ ID NO: 52, an HCDR2 set forth in SEQ ID NO: 53, and an HCDR3 set forth in SEQ ID NO: 54; an LCDR1 set forth in SEQ ID NO: 55, an LCDR2 set forth in SEQ ID NO: 56, and an LCDR3 set forth in SEQ ID NO: 57.

In some embodiments, the isolated antibody or the antigen-binding fragment specifically binding to the Nectin-4 protein of the present disclosure comprises:
(a) a heavy chain variable region and a light chain variable region, wherein the heavy chain variable region comprises the sequence of SEQ ID NO: 1 or a sequence having at least 90%, 91%, 92%, 93%, 94%, 95%, 96%, 97%, 98%, or 99% identity thereto, and the light chain variable region comprises the sequence of SEQ ID NO: 2 or a sequence having at least 90%, 91%, 92%, 93%, 94%, 95%, 96%, 97%, 98%, or 99% identity thereto;
   for example, a heavy chain variable region set forth in SEQ ID NO: 1 and a light chain variable region set forth in SEQ ID NO: 2;
   a heavy chain variable region set forth in SEQ ID NO: 18 and a light chain variable region set forth in SEQ ID NO: 20;
   a heavy chain variable region set forth in SEQ ID NO: 18 and a light chain variable region set forth in SEQ ID NO: 21;
   a heavy chain variable region set forth in SEQ ID NO: 18 and a light chain variable region set forth in SEQ ID NO: 22;
   a heavy chain variable region set forth in SEQ ID NO: 18 and a light chain variable region set forth in SEQ ID NO: 23;
   a heavy chain variable region set forth in SEQ ID NO: 18 and a light chain variable region set forth in SEQ ID NO: 24;
   a heavy chain variable region set forth in SEQ ID NO: 19 and a light chain variable region set forth in SEQ ID NO: 20;
   a heavy chain variable region set forth in SEQ ID NO: 19 and a light chain variable region set forth in SEQ ID NO: 21;
   a heavy chain variable region set forth in SEQ ID NO: 19 and a light chain variable region set forth in SEQ ID NO: 22;
   a heavy chain variable region set forth in SEQ ID NO: 19 and a light chain variable region set forth in SEQ ID NO: 23;
   a heavy chain variable region set forth in SEQ ID NO: 19 and a light chain variable region set forth in SEQ ID NO: 24;
(b) a heavy chain variable region and a light chain variable region, wherein the heavy chain variable region comprises the sequence of SEQ ID NO: 3 or a sequence having at least 90%, 91%, 92%, 93%, 94%, 95%, 96%, 97%, 98%, or 99% identity thereto, and the light chain variable region comprises the sequence of SEQ ID NO: 4 or a sequence having at least 90%, 91%, 92%, 93%, 94%, 95%, 96%, 97%, 98%, or 99% identity thereto;
   for example, a heavy chain variable region set forth in SEQ ID NO: 3 and a light chain variable region set forth in SEQ ID NO: 4; or
(c) a heavy chain variable region and a light chain variable region, wherein the heavy chain variable region comprises the sequence of SEQ ID NO: 5 or a sequence having at least 90%, 91%, 92%, 93%, 94%, 95%, 96%, 97%, 98%, or 99% identity thereto, and the light chain variable region comprises the sequence of SEQ ID NO: 6 or a sequence having at least 90%, 91%, 92%, 93%, 94%, 95%, 96%, 97%, 98%, or 99% identity thereto;
   for example, a heavy chain variable region set forth in SEQ ID NO: 5 and a light chain variable region set forth in SEQ ID NO: 6.

In some embodiments, the isolated antibody or the antigen-binding fragment specifically binding to the Nectin-4 protein of the present disclosure comprises:
(a) SEQ ID NO: 11 or a heavy chain sequence having at least 90%, 91%, 92%, 93%, 94%, 95%, 96%, 97%, 98%, or 99% identity thereto, and SEQ ID NO: 12 or a light chain sequence having at least 90%, 91%, 92%, 93%, 94%, 95%, 96%, 97%, 98%, or 99% identity thereto;
   for example, the heavy chain sequence set forth in SEQ ID NO: 11 and the light chain sequence set forth in SEQ ID NO: 12;
   the heavy chain sequence set forth in SEQ ID NO: 25 and the light chain sequence set forth in SEQ ID NO: 27;
   the heavy chain sequence set forth in SEQ ID NO: 25 and the light chain sequence set forth in SEQ ID NO: 28;
   the heavy chain sequence set forth in SEQ ID NO: 25 and the light chain sequence set forth in SEQ ID NO: 29;
   the heavy chain sequence set forth in SEQ ID NO: 25 and the light chain sequence set forth in SEQ ID NO: 30;
   the heavy chain sequence set forth in SEQ ID NO: 25 and the light chain sequence set forth in SEQ ID NO: 31;
   the heavy chain sequence set forth in SEQ ID NO: 26 and the light chain sequence set forth in SEQ ID NO: 27;
   the heavy chain sequence set forth in SEQ ID NO: 26 and the light chain sequence set forth in SEQ ID NO: 28;
   the heavy chain sequence set forth in SEQ ID NO: 26 and the light chain sequence set forth in SEQ ID NO: 29;
   the heavy chain sequence set forth in SEQ ID NO: 26 and the light chain sequence set forth in SEQ ID NO: 30;
   the heavy chain sequence set forth in SEQ ID NO: 26 and the light chain sequence set forth in SEQ ID NO: 31;
(b) SEQ ID NO: 13 or a heavy chain sequence having at least 90%, 91%, 92%, 93%, 94%, 95%, 96%, 97%, 98%, or 99% identity thereto, and SEQ ID NO: 14 or a light chain sequence having at least 90%, 91%, 92%, 93%, 94%, 95%, 96%, 97%, 98%, or 99% identity thereto; or
(c) SEQ ID NO: 15 or a heavy chain sequence having at least 90%, 91%, 92%, 93%, 94%, 95%, 96%, 97%, 98%, or 99% identity thereto, and SEQ ID NO: 16 or a light chain sequence having at least 90%, 91%, 92%, 93%, 94%, 95%, 96%, 97%, 98%, or 99% identity thereto.

In some embodiments, the antigen-binding fragment of the isolated anti-Nectin-4 antibody of the present disclosure is a Fab, a Fab', a F(ab')2, an Fv, a single-chain Fv, or a single-chain Fab.

In some embodiments, the isolated antibody or the antigen-binding fragment specifically binding to the Nectin-4 protein of the present disclosure is a fully human antibody.

In some embodiments, the isolated antibody or the antigen-binding fragment specifically binding to the Nectin-4 protein of the present disclosure is an IgG antibody, preferably a human IgG antibody, and more preferably a human IgG1 or human IgG4 antibody.

In a second aspect, the present disclosure provides a multispecific antibody comprising an isolated antibody or an antigen-binding fragment specifically binding to a Nectin-4 protein, e.g., comprising the isolated antibody or the antigen-binding fragment specifically binding to the Nectin-4 protein according to the first aspect of the present disclosure. Preferably, the multispecific antibody is a trispecific antibody or a bispecific antibody.

In some embodiments, the present disclosure provides a bispecific antibody comprising an isolated antibody or an antigen-binding fragment specifically binding to a Nectin-4 protein and an isolated antibody or an antigen-binding fragment specifically binding to a T cell co-stimulatory receptor (e.g., ICOS, 4-1BB, CD28, or CD86). For example, the bispecific antibody comprises the isolated antibody or the antigen-binding fragment specifically binding to the Nectin-4 protein according to the first aspect of the present disclosure and an isolated antibody or an antigen-binding fragment specifically binding to a T cell co-stimulatory receptor (e.g., ICOS, 4-1BB, CD28, or CD86).

In some embodiments, the present disclosure provides a bispecific antibody comprising, from the amino-terminus to the carboxyl-terminus:
(a) a first moiety, which is an isolated antibody or an antigen-binding fragment specifically binding to a Nectin-4 protein, e.g., the isolated antibody or the antigen-binding fragment specifically binding to the Nectin-4 protein according to the first aspect of the present disclosure, and
(b) a second moiety, which is an isolated antibody or an antigen-binding fragment specifically binding to a T cell co-stimulatory receptor 4-1BB;
wherein the antigen-binding fragments in the first moiety and the second moiety are a Fab, a Fab', a F(ab')2, an Fv, a single-chain Fv, or a single-chain Fab.

In some embodiments, the present disclosure provides a bispecific antibody, which is an anti-Nectin-4/4-1BB bispecific antibody comprising:
(a) a first moiety, which is an isolated antibody or an antigen-binding fragment specifically binding to a Nectin-4 protein, and
(b) a second moiety, which is an isolated antibody or an antigen-binding fragment specifically binding to a T cell co-stimulatory receptor 4-1BB; and
the bispecific antibody is a bispecific antibody with high affinity for Nectin-4 and low affinity for 4-1BB, a K_{D} value of the bispecific antibody for targeting a human Nectin-4 protein is two orders of magnitude higher than a K_{D} value of the bispecific antibody for targeting a human 4-1BB protein, and the high affinity of the bispecific antibody for human Nectin-4 can effectively reduce the side effects of targeting human 4-1BB. By targeting Nectin-4 and the co-stimulatory receptor 4-1BB, the anti-Nectin-4/4-1BB bispecific antibody can not only block the binding of Nectin-4 to TIGIT, but also promote the effective tumor-directed immune T cell activation and killing by binding to the T cell co-stimulatory receptor 4-1BB.

In some embodiments, the antigen-binding fragment of the isolated antibody specifically binding to 4-1BB of the present disclosure is a Fab, a Fab', a F(ab')2, an Fv, a single-chain Fv, or a single-chain Fab.

In a third aspect, the present disclosure provides a nucleic acid encoding the isolated antibody or the antigen-binding fragment specifically binding to the Nectin-4 protein according to the first aspect described above or encoding the multispecific antibody according to the second aspect described above, a vector (preferably, an expression vector) comprising the nucleic acid, and a host cell comprising the nucleic acid or the vector. In some embodiments, the host cell is prokaryotic or eukaryotic, e.g., selected from an *Escherichia coli* cell, a yeast cell, a mammalian cell, or other cells suitable for preparing the antibody or the antigen-binding fragment, or the multispecific antibody. In some embodiments, the host cell is an HEK 293 cell or a CHO cell.

In a fourth aspect, the present disclosure provides a method for preparing the isolated antibody or the antigen-binding fragment specifically binding to the Nectin-4 protein according to the first aspect described above or the multispecific antibody according to the second aspect described above, which comprises culturing the host cell of the present disclosure under conditions suitable for expressing a nucleic acid encoding the isolated antibody or the antigen-binding fragment specifically binding to the Nectin-4 protein according to the first aspect described above or encoding the multispecific antibody according to the second aspect described above, and optionally recovering the isolated antibody or the antigen-binding fragment specifically binding to the Nectin-4 protein according to the first aspect described above or the multispecific antibody according to the second aspect described above from the host cell or a culture medium.

In a fifth aspect, the present disclosure provides a pharmaceutical composition comprising the isolated antibody or the antigen-binding fragment specifically binding to the Nectin-4 protein according to the first aspect described above or the multispecific antibody according to the second aspect described above, and a pharmaceutically acceptable carrier.

In some embodiments, the pharmaceutical composition of the present disclosure comprises the isolated antibody or the antigen-binding fragment specifically binding to the Nectin-4 protein according to the first aspect described above or the multispecific antibody according to the second aspect described above, and comprises an additional therapeutic agent selected from an oncolytic drug, a cytotoxic agent, a cytokine, and an inhibitor of other immune checkpoint molecules (e.g., PD-1), and a pharmaceutically acceptable carrier.

In a sixth aspect, the present disclosure provides use of the isolated antibody or the antigen-binding fragment specifically binding to the Nectin-4 protein according to the first aspect described above or the multispecific antibody according to the second aspect described above in preparing a medicament for diagnosing, preventing, and/or treating a disease associated with high Nectin-4 expression; for example, the disease is a cancer. In some embodiments, the cancer is a solid tumor or a hematological cancer; for example, the cancer is selected from colon cancer, rectal cancer, colorectal cancer, esophageal cancer, skin cancer, urothelial carcinoma, ovarian cancer, pancreatic cancer, bladder cancer, non-Hodgkin's lymphoma, Hodgkin's lymphoma, acute lymphocytic leukemia, multiple myeloma, breast cancer, gastric cancer, hepatocellular carcinoma, non-small cell lung cancer, small cell lung cancer, melanoma, glioblastoma, renal cell carcinoma, and prostate cancer.

In a seventh aspect, the present disclosure provides a method for preventing and/or treating a disease, which comprises administering to a subject in need thereof an effective amount of the isolated antibody or the antigen-binding fragment specifically binding to the Nectin-4 protein of the present disclosure, the multispecific antibody of the present disclosure, the nucleic acid of the present disclosure, the vector of the present disclosure, or the host cell of the present disclosure, wherein the subject is a mammal; preferably, the subject is a human; the disease is a disease associated with high Nectin-4 expression; for example, the disease is a cancer. In some embodiments, the cancer is a solid tumor or a hematological cancer; for example, the cancer is selected from colon cancer, rectal cancer, colorectal cancer, esophageal cancer, skin cancer, urothelial carcinoma, ovarian cancer, pancreatic cancer, bladder cancer, non-Hodgkin's lymphoma, Hodgkin's lymphoma, acute lymphocytic leukemia, multiple myeloma, breast cancer, gastric cancer, hepatocellular carcinoma, non-small cell lung cancer, small cell lung cancer, melanoma, glioblastoma, renal cell carcinoma, and prostate cancer.

In an eighth aspect, the present disclosure provides a method for detecting the presence or level of a Nectin-4 protein in a sample, which comprises contacting the sample with the isolated antibody or the antigen-binding fragment specifically binding to the Nectin-4 protein of the present disclosure under conditions allowing formation of a complex between the antibody or the antigen-binding fragment thereof and the Nectin-4 protein, and detecting the formation of the complex, wherein, for example, the detection method is a detection method for non-diagnostic purposes.

### BRIEF DESCRIPTION OF THE DRAWINGS

The preferred embodiments of the present disclosure described in detail below will be better understood when read in conjunction with the following drawings. For the purpose of illustrating the present disclosure, currently preferred embodiments are shown in the drawings. However, it should be understood that the present disclosure is not limited to the precise arrangement and means of the embodiments shown in the drawings.
FIG. 1A shows the detection results of the binding ability of murine chimeric anti-Nectin-4 antibodies (i.e., antibody 23-H4F3, antibody 6-B9B6, and antibody 32-A12C7) to tumor cells HT-1376 expressing human Nectin-4 at the cellular level.
FIG. 1B shows the detection results of the binding ability of murine chimeric anti-Nectin-4 antibodies (i.e., antibody 23-H4F3, antibody 6-B9B6, and antibody 32-A12C7) to cells CHO-K1/cynoNectin4 expressing the cynomolgus monkey Nectin-4 protein at the cellular level.
FIGs. 2A and 2B show the detection results of the binding ability of murine chimeric anti-Nectin-4 antibodies 23-H4F3 and 6-B9B6 to Nectin family proteins Nectin-1, Nectin-2, Nectin-3, and Nectin-4, respectively.
FIGs. 3A and 3B show the detection results of the binding ability of humanized anti-Nectin-4 antibodies (i.e., humanized antibodies huH4F3-1 to huH4F3-10) to tumor cells HT-1376 expressing human Nectin-4 and cells CHO-K1/cynoNectin4 expressing the cynomolgus monkey Nectin-4 protein at the cellular level, respectively.
FIG. 4A shows the detection results of the activation of the NF-κB-Luc/4-1BB reporter gene cell line by anti-Nectin-4×4-1BB bispecific antibodies in the presence of tumor cells HT-1376 expressing Nectin-4. In the figure, the abscissa represents the antibody concentration, and the ordinate represents the relative light unit (RLU) value.
FIG. 4B shows the detection results of the activation of the NF-κB-Luc/4-1BB reporter gene cell line by anti-Nectin-4×4-1BB bispecific antibodies in the presence of cells 293T not expressing Nectin-4. In the figure, the abscissa represents the antibody concentration, and the ordinate represents the RLU value.
FIG. 5 shows the changes in tumor volume of mice after administration of anti-Nectin-4×4-1BB bispecific antibodies to tumor-bearing mice. In the figure, the abscissa represents the days after grouping of tumor-bearing mice.
FIG. 6 shows the changes in body weight of mice after administration of anti-Nectin-4×4-1BB bispecific antibodies to tumor-bearing mice. In the figure, the abscissa represents the days after grouping of tumor-bearing mice.

### DETAILED DESCRIPTION

Before the present disclosure is described in detail, it should be understood that the present disclosure is not limited to the particular methods or experimental conditions described in this description, as the methods and conditions may vary. In addition, the terms used herein are for the purpose of describing particular embodiments only and are not intended to be limiting.

### I. Definitions

Unless defined otherwise, all technical and scientific terms used herein have the same meaning as commonly understood by those of ordinary skill in the art. For the purposes of the present disclosure, the following terms are defined below.

The term "about" used in combination with a numerical value is intended to encompass the numerical values in a range from a lower limit less than the specified numerical value by 10% to an upper limit greater than the specified numerical value by 10%.

The term "and/or", when used to connect two or more options, should be understood to refer to any one of the options or any two or more of the options.

As used herein, the term "comprise" or "include" is intended to refer to the inclusion of the elements, integers, or steps, but not the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

The term "antibody" is used herein in the broadest sense and includes, but is not limited to, monoclonal antibodies, polyclonal antibodies, and multispecific antibodies (e.g., bispecific antibodies), so long as they exhibit the desired antigen-binding activity. The antibody can be an intact antibody (e.g., having two full-length light chains and two full-length heavy chains) of any type and subtype (e.g., IgM, IgD, IgG1, IgG2, IgG3, IgG4, IgE, IgA1, and IgA2). A monomer of an intact antibody is a four-peptide-chain molecule formed by two full-length light chains and two full-length heavy chains linked via disulfide bonds, also referred to as the monomer of an Ig molecule. An antibody monomer is the basic structure that constitutes an antibody.

"Isolated antibody" used herein refers to an antibody that is substantially free of other antibodies (Abs) with different antigenic specificities (e.g., an isolated antibody or an antigen-binding fragment thereof specifically binding to a Nectin-4 protein is substantially free of Abs specifically binding to antigens other than the Nectin-4 protein). In certain embodiments, the antibody is purified to a purity greater than 80% or 90%, preferably greater than 95% or 99%, as determined by, for example, electrophoresis (e.g., SDS-PAGE, isoelectric focusing (IEF), or capillary electrophoresis), or chromatography (e.g., ion exchange or reverse-phase HPLC).

"Epitope" or "antigenic determinant" refers to an antigenic determinant that interacts with a specific antigen-binding site referred to as a paratope in the variable region of an antibody molecule. A single antigen may have one or more epitopes. Thus, different antibodies may bind to different regions on the antigen and may have different biological effects. An epitope can be formed from contiguous amino acids or noncontiguous amino acids juxtaposed via tertiary folding of a protein. Epitopes formed from contiguous amino acids are generally retained on exposure to denaturing solvents, whereas epitopes formed by tertiary folding are generally lost on treatment with denaturing solvents. An epitope generally comprises at least 3, and more generally at least 5, about 9, or about 8-10 amino acids in a unique spatial conformation.

The term "antigen-binding fragment" is a portion or segment of an intact antibody or a complete antibody that has fewer amino acid residues than the intact antibody or the complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragment includes, but is not limited to, a Fab, a Fab', a F(ab')₂, an Fv, a single-chain Fv (scFv), a single-chain Fab, a diabody, a single-domain antibody (sdAb, a nanobody), a camelid Ig, an Ig NAR, a F(ab)'₃ fragment, a bis-scFv, an (scFv)₂, a minibody, a bifunctional antibody, a trifunctional antibody, a tetrafunctional antibody, and a disulfide-stabilized Fv protein ("dsFv"). The term also includes genetically engineered forms, such as chimeric antibodies (e.g., humanized murine antibodies), heteroconjugate antibodies (e.g., bispecific antibodies), and antigen-binding fragments thereof. For a more detailed description, see also: Pierce Catalog and Handbook, 1994-1995 (PierceChemical Co., Rockford, Illinois (IL)); Kuby, Immunology, 3rd Ed., W.H. Freeman&Co., New York, 1997.

The terms "full antibody", "full-length antibody", "complete antibody", and "intact antibody" are used interchangeably herein to refer to a glycoprotein comprising at least two heavy chains (HCs) and two light chains (LCs) interconnected by disulfide bonds. Each heavy chain consists of a heavy chain variable region (abbreviated herein as VH) and a heavy chain constant region. The heavy chain constant region consists of 3 domains, CH1, CH2, and CH3. Each light chain consists of a light chain variable region (abbreviated herein as VL) and a light chain constant region. The light chain constant region consists of one domain CL. Mammalian heavy chains are classified as α, δ, ε, y, and µ. Mammalian light chains are classified as λ or κ. Immunoglobulins comprising α, δ, ε, γ, and µ heavy chains are classified as immunoglobulins (Ig)A, IgD, IgE, IgG, and IgM. The complete antibody forms a "Y" shape. The stem of Y consists of the second and third constant regions of the two heavy chains (and, for IgE and IgM, the fourth constant region) bound together, and disulfide bonds (interchain) are formed in the hinge. Heavy chains γ, α, and δ have a constant region consisting of three tandem (in a line) Ig domains and a hinge region for increasing flexibility; heavy chains µ and ε have a constant region consisting of four immunoglobulin domains. The second and third constant regions are referred to as the "CH2 domain" and "CH3 domain", respectively. Each arm of Y comprises the variable region and the first constant region of a single heavy chain bound to the variable and constant regions of a single light chain. The variable regions of the light and heavy chains are responsible for antigen binding. The light chain variable region and the heavy chain variable region each comprise a "framework" region interspersed with three hypervariable regions (also known as "complementarity determining regions" or "CDRs"). "Complementarity determining region", "CDR region", "CDR", or "highly variable region" (used interchangeably herein with hypervariable region "HVR") is a region in an antibody variable domain that is highly variable in sequence and forms a structurally defined loop ("hypervariable loop") and/or comprises antigen-contacting residues ("antigen-contacting sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR numbering systems including, e.g., Chothia based on the three-dimensional structure of antibodies and the topology of the CDR loops (Chothia et al., (1989) Nature, 342: 877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

However, it should be noted that the boundaries of the CDRs of the variable regions of the same antibody obtained on the basis of different numbering systems may differ. That is, the CDR sequences of the variable regions of the same antibody defined under different numbering systems are different. Therefore, when it comes to defining an antibody with specific CDR sequences defined in the present disclosure, the scope of the antibody also encompasses such antibodies whose variable region sequences comprise the specific CDR sequences, but have claimed CDR boundaries different from the specific CDR boundaries defined by the present disclosure due to different schemes (e.g., different numbering system rules or their combinations) applied.

The boundaries of the CDRs of the antibodies of the present disclosure can be determined by manual evaluation according to any scheme in the art or a combination thereof. Unless otherwise stated, the term "CDR" or "CDR sequence" used in the present disclosure encompasses CDR sequences determined by any one of the schemes described above.

The sequences of the framework regions of the different light or heavy chains are relatively conserved within a species (e.g., human). The framework regions of the antibody, which are the combined framework regions of the component light and heavy chains, are used to locate and align the CDRs in the three-dimensional space. The CDRs are primarily responsible for binding to an epitope of an antigen. Antibodies with different specificities (i.e., different combining sites for different antigens) have different CDRs. Although the CDRs differ from antibody to antibody, only a limited number of amino acid positions within the CDRs are directly involved in antigen binding. These positions within the CDRs are called specificity determining residues (SDRs).

"Monoclonal antibody" is an antibody produced by a single clone of B lymphocytes or by cells into which the light and heavy chain genes of a single antibody have been transfected. Monoclonal antibodies are produced by methods known to those skilled in the art, for example, by preparing hybrid antibody-forming cells from fusions of myeloma cells with immune spleen cells. Monoclonal antibodies include humanized monoclonal antibodies.

"Fv" is the minimum antibody fragment that contains a complete antigen-binding site. In one embodiment, a double-chain Fv consists of one heavy chain variable domain and one light chain variable domain in a tight, non-covalently associated dimer. In a single-chain Fv (scFv), one heavy chain variable domain and one light chain variable domain can be covalently linked via a flexible peptide linker, such that the light chain and heavy chain can be associated in a "dimeric" structure similar to that of the double-chain Fv. In this configuration, the three hypervariable regions (HVRs) of each variable domain interact to define an antigen-binding site on the surface of the VH-VL dimer. The six HVRs collectively confer antigen-binding specificity to the antibody. Unless otherwise indicated, as used herein, the scFv may have a VL variable region and a VH variable region in any order (e.g., relative to the N-terminus and the C-terminus of the polypeptide), and may comprise VL-linker-VH or VH-linker-VL.

An Fab fragment contains a heavy chain variable domain and a light chain variable domain and also contains the constant domain of the light chain and the first constant domain (CH1) of the heavy chain. Fab' fragments differ from Fab fragments by the addition of a few residues to the carboxyl-terminus of the heavy chain CH1 domain, including one or more cysteines from an antibody hinge region. Fab'-SH is the name used herein for Fab', wherein the cysteine residue of the constant domain carries a free thiol group. F(ab')2 antibody fragments were originally produced as pairs of Fab' fragments with hinge cysteines between them. Other chemical conjugations of antibody fragments are also known.

The term "chimeric antibody" refers to an antibody in which (a) a constant region or a portion thereof is modified, substituted, or exchanged such that antigen-binding sites are linked to constant regions of different or modified classes and/or species, etc.; or (b) a variable region or a portion thereof is modified, substituted, or exchanged by variable regions with different or modified antigenic specificities. In some embodiments of the present disclosure, the constant region of a murine antibody is modified by substituting it with a constant region from a human immunoglobulin. Due to the substitution with a human constant region, the chimeric antibody may retain its specificity for recognizing antigens, while having reduced antigenicity in humans as compared with the original murine antibody.

"Humanized" antibody refers to a chimeric antibody comprising amino acid residues from non-human CDRs and amino acid residues from human FRs. In some embodiments, all or substantially all CDRs in the humanized antibody correspond to CDRs of a non-human antibody, and all or substantially all FRs correspond to FRs of a human antibody. The humanized antibody may optionally comprise at least a portion of an antibody constant region from a human antibody. The "humanized form" of an antibody (e.g., a non-human antibody) refers to an antibody that has been humanized. In some embodiments, the amino acid residues of the CDRs of the humanized antibody do not precisely correspond to the amino acid residues of the murine CDRs as parents. For example, mutations are performed at specific positions of the CDRs for high-affinity contact with the target antigen.

"Fully human antibody" or "human antibody" refers to any antibody whose variable and constant region sequences are all human sequences, without any non-human antibody sequences. The term also encompasses antibodies recombinantly produced in non-human cells, which may additionally bear glycosylation atypical of human cells.

The term "multispecific antibody" refers to an antibody having binding specificities for a plurality of different epitopes. The epitopes of the multispecific antibody may be from the same antigen or from a plurality of different antigens. The term "bispecific antibody" refers to an antibody having binding specificities for two different epitopes. The epitopes of the bispecific antibody may be from the same antigen or from two different antigens.

The term "tumor antigen" includes tumor-associated antigens (TAAs) and tumor-specific antigens (TSAs). The term "tumor-associated antigen" (TAA) refers to a protein present on tumor cells, as well as on normal cells in selected organs during the fetus (once embryonic antigens) and after birth, but at concentrations far lower than those in tumor cells. Tumor-associated antigens may also be present in the stroma adjacent to tumor cells, but are expressed in lower amounts in the stroma elsewhere in the body. The term "tumor-specific antigen" (TSA) refers to an antigen that is present predominantly on tumor cells of a mammalian subject but is generally not found on normal cells of a mammalian subject.

The terms "Nectin protein family" and "Nectin family" are used interchangeably and refer to cell adhesion molecules that form a physical connection between adjacent cells to achieve intercellular communication, migration, and other important cellular processes. The Nectin protein family includes at least 4 types of human Nectin proteins, namely a Nectin-1 protein, a Nectin-2 protein, a Nectin-3 protein, and a Nectin-4 protein, wherein the expression of the Nectin-4 protein in healthy humans is mainly limited to placental and embryonic tissues. Compared with healthy adult tissues, many types of tumor cells have high expression of the Nectin-4 protein. The Nectin-4 protein is a tumor-associated antigen.

The term "specific binding" or "binding" used when referring to an antigen and an antibody means that the antibody forms a complex with the antigen that is relatively stable under physiological conditions. Methods for determining whether an antibody specifically binds to an antigen are well known in the art and include, for example, the surface plasmon resonance assay, the MSD assay (Estep, P. et al., High throughput solution-based measurement of antibody-antigen affinity and epitope binning, MAbs, 2013. 5(2): p.270-278), the ForteBio affinity assay (Estep, P et al., High throughput solution Based measurement of antibody-antigen affinity and epitope binning. MAbs, 2013.5(2): p. 270-8).

"Affinity" refers to the strength of the sum of all non-covalent interactions between a single binding site of a molecule (e.g., an antibody) and its binding partner (e.g., an antigen). Unless otherwise stated, when used herein, "binding affinity" refers to the intrinsic binding affinity that reflects a 1:1 interaction between members of a binding pair (e.g., an antibody and an antigen). The affinity of a molecule X for its partner Y can generally be represented by a binding dissociation equilibrium constant (K_{D}). Affinity can be measured by common methods known in the art, including those known in the prior art and described herein.

As used herein, the term "variant" refers to a heavy chain variable region or a light chain variable region that has been modified by at least one, e.g., 1, 2, or 3 amino acid substitutions, deletions, or additions, wherein the modified antigen-binding protein comprising the heavy chain or light chain variant substantially retains the biological characteristics of the antigen-binding protein prior to modification. In one embodiment, an antigen-binding protein comprising a variant heavy chain variable region or light chain variable region sequence retains 60%, 70%, 80%, 90%, or 100% of the biological characteristics of the antigen-binding protein prior to modification. It should be understood that each heavy chain variable region or light chain variable region may be modified individually or in combination with another heavy chain variable region or light chain variable region. The antigen-binding protein of the present disclosure comprises an amino acid sequence of a heavy chain variable region having 90%, 91%, 92%, 93%, 94%, 95%, 96%, 97%, 98%, or 99% homology to the amino acid sequence of the heavy chain variable region described herein. The antigen-binding protein of the present disclosure comprises an amino acid sequence of a light chain variable region having 90%, 91%, 92%, 93%, 94%, 95%, 96%, 97%, 98%, or 99% homology to the amino acid sequence of the light chain variable region described herein. The percent homology may be over the entire heavy chain variable region and/or the entire light chain variable region, or the percent homology may be limited to the framework region, while the sequences corresponding to the CDRs have 100% identity to the CDRs disclosed herein within the heavy chain variable region and/or the light chain variable region. As used herein, the term "CDR variant" refers to a CDR that has been modified by at least one, e.g., 1, 2, or 3 amino acid substitutions, deletions, or additions, wherein the modified antigen-binding protein comprising the CDR variant substantially retains the biological characteristics of the antigen-binding protein prior to modification. In one embodiment, an antigen-binding protein comprising a variant CDR retains 60%, 70%, 80%, 90%, or 100% of the biological characteristics of the antigen-binding protein prior to modification. It should be understood that each CDR that can be modified may be modified individually or in combination with another CDR. In one embodiment, the modification is a substitution, particularly a conservative substitution.

"Polynucleotide" and "nucleic acid" are used interchangeably herein and refer to a nucleotide chain of any length, including a DNA and an RNA. The nucleotides may be deoxyribonucleotides, ribonucleotides, modified nucleotides or bases, and/or their analogs, or any substrate capable of being incorporated into a strand by a DNA or RNA polymerase.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison purposes). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate the percent identity between the sequences. In one preferred embodiment, the percent identity between two amino acid sequences is determined with the Needlema and Wunsch ((1970) J. Mol. Biol. 48: 444-453) algorithm integrated in the GAP program of the GCG software package (available at http://www.gcg.com), using the Blossum 62 matrix or PAM250 matrix, a gap weight of 16, 14, 12, 10, 8, 6, or 4, and a length weight of 1, 2, 3, 4, 5, or 6. In another preferred embodiment, the percent identity between two nucleotide sequences is determined with the GAP program in the GCG software package (available at http://www.gcg.com), using the NWSgapdna.CMP matrix, a gap weight of 40, 50, 60, 70, or 80, and a length weight of 1, 2, 3, 4, 5, or 6. A particularly preferred parameter set (and one that should be used unless otherwise stated) is the Blossum 62 scoring matrix with a gap penalty of 12, a gap extension penalty of 4, and a frameshift gap penalty of 5.

The percent identity between two amino acid sequences or nucleotide sequences can also be determined with the E. Meyers and W. Miller algorithm ((1989) CABIOS, 4:11-17) incorporated into the ALIGN program (version 2.0), using the PAM120 weighted remainder table, a gap length penalty of 12, and a gap penalty of 4.

Additionally or alternatively, the nucleic acid sequences and protein sequences described herein can further be used as "query sequences" to perform searches against public databases, for example, to identify other family member sequences or related sequences.

For polypeptide sequences, "conservative modifications" include substitutions of, deletions of, or additions to a polypeptide sequence that result in the substitution of a certain amino acid with a chemically similar amino acid. Conservative substitution tables providing functionally similar amino acids are well known in the art. Such conservatively modified variants are in addition to and do not exclude the polymorphic variants, interspecies homologs, and alleles of the present disclosure. The following 8 groups contain amino acids that are conservative substitutions of each other: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M) (see, e.g., Creighton, Proteins (1984)). In some embodiments, the term "conservative sequence modification" is used to refer to an amino acid modification that does not significantly affect or alter the binding characteristics of an antibody comprising the amino acid sequence.

The term "immune checkpoint" refers to a class of inhibitory signaling molecules present in the immune system that avoid tissue damage by regulating the persistence and strength of the immune response in peripheral tissues and participate in the maintenance of tolerance to self-antigens (Pardoll DM., The blockade of immune checkpoints in cancer immunotherapy. Nat Rev Cancer, 2012, 12(4): 252-264). Studies have found that one of the reasons that tumor cells can escape the immune system in the body and proliferate out of control is that they take advantage of the inhibitory signaling pathway of immune checkpoints, thereby inhibiting the activity of T lymphocytes, which makes T lymphocytes unable to effectively exert the killing effect on tumors (Yao S, Zhu Y, and Chen L., Advances in targeting cell surface signaling molecules for immune modulation. Nat Rev Drug Discov, 2013, 12(2): 130-146). Immune checkpoint molecules include, but are not limited to, programmed death 1 (PD-1), PD-L1, PD-L2, cytotoxic T lymphocyte antigen 4 (CTLA-4), LAG-3, TIM-3, and a protein with T cell immunoglobulin and ITIM domains (TIGIT).

The terms "co-stimulatory molecule" and "co-stimulatory receptor" are used interchangeably and refer to a corresponding binding partner on a lymphocyte that specifically binds to a co-stimulatory ligand to mediate a co-stimulatory response (e.g., but not limited to, proliferation) of the lymphocyte. Co-stimulatory molecules are cell surface molecules other than antigen receptors or ligands thereof that are required for an effective response of lymphocytes to antigens. Co-stimulatory molecules include, but are not limited to, MHC class I molecules, TNF receptor proteins, cytokine receptors, integrins, signaling lymphocytic activation molecules (SLAM proteins), activated NK cell receptors, inducible co-stimulators (ICOS) (i.e., CD278), OX40, CD40, GITR, 4-1BB (i.e., CD137), CD27, CD28, and CD86. In some embodiments, the "co-stimulatory molecule" is ICOS, 4-1BB, CD28, and/or CD86.

As used herein, "vector" refers to a construct capable of delivering one or more genes or sequences of interest into a host cell and preferably expressing the genes or sequences in the host cell. Examples of vectors include, but are not limited to, viral vectors, naked DNA or RNA expression vectors, plasmid, cosmid, or phage vectors, DNA or RNA expression vectors associated with cationic condensing agents, DNA or RNA expression vectors encapsulated in liposomes, and certain eukaryotic cells, such as producer cells.

The terms "host cell", "host cell line", and "host cell culture" are used interchangeably in the present disclosure and refer to cells into which exogenous nucleic acids have been introduced, including progeny of such cells. Host cells include "transformants" and "transformed cells", which include primary transformed cells and progeny derived therefrom, regardless of the number of passages. The progeny may not be exactly the same as parent cells in terms of the nucleic acid content, but may contain mutations. Mutant progeny that have the same function or biological activity as the cells screened or selected from the initially transformed cells are included herein.

The present disclosure also relates to a method for producing a monoclonal antibody, which comprises culturing the host cell described in the present disclosure to produce the monoclonal antibody described in the present disclosure above.

As used herein, "subject" or "individual" refers to an animal, preferably a mammal, and more preferably a human, in need of amelioration, prevention, and/or treatment of a disease or disorder, such as a viral infection. Mammals also include, but are not limited to, farm animals, racing animals, pets, primates, horses, dogs, cats, mice, and rats. The term encompasses human subjects having a disease or at risk of having a disease. In the present disclosure, administering the antibody described in the present disclosure or the pharmaceutical composition or product described in the present disclosure to a subject in need thereof refers to administering an effective amount of the antibody or the pharmaceutical composition or product, etc.

As used in the present disclosure, the term "effective amount" means an amount of a drug or pharmaceutical preparation that elicits the biological or pharmaceutical response in a tissue, system, animal, or human that is being sought, for example, by a researcher or clinician. In addition, the term "therapeutically effective amount" means an amount that causes improved treatment, cure, prevention, or alleviation of a disease, disorder, or side effect, or an amount that causes a reduction in the rate of progression of a disease or condition, as compared with a corresponding subject that does not receive that amount. The term also encompasses within its scope an amount effective to enhance normal physiological functions.

### II. Isolated Anti-Nectin-4 Antibody of the Present Disclosure

The present disclosure provides an isolated antibody against Nectin-4 and an antigen-binding fragment thereof. The protein or the fragment thereof used as a sensitized antigen for obtaining the isolated antibody against Nectin-4 of the present disclosure is preferably a Nectin-4 protein from a human or an extracellular portion thereof. The base sequences and amino acid sequences of human Nectin-4 and homologs thereof can be obtained, for example, by accessing GenBank (NCBI, USA) (e.g., NCBI accession No. NP_112178.2). In one embodiment, the extracellular region of a human Nectin-4 protein has the amino acid sequence set forth in SEQ ID NO: 39. The terms "antibody binding to a Nectin-4 protein", "antibody binding to Nectin-4", "anti-Nectin-4 protein antibody", "anti-Nectin-4 antibody", "isolated antibody binding to a Nectin-4 protein", and "Nectin-4 protein antibody" are used interchangeably herein and refer to an antibody of the present disclosure that is capable of binding to a Nectin-4 protein with sufficient affinity such that the antibody can be used as a diagnostic agent, a prophylactic agent, and/or a therapeutic agent targeting the Nectin-4 protein.

The isolated anti-Nectin-4 antibody and the antigen-binding fragment specifically binding to the Nectin-4 protein with high affinity of the present disclosure comprise:
(a) 3 CDRs in the amino acid sequence of a heavy chain variable region set forth in SEQ ID NO: 1 and 3 CDRs in the amino acid sequence of a light chain variable region set forth in SEQ ID NO: 2; or a variant with one or more CDRs each having no more than 3 amino acid modifications relative to the 6 CDR regions described above;
   for example, 3 CDRs in the amino acid sequence of a heavy chain variable region set forth in SEQ ID NO: 18 and 3 CDRs in the amino acid sequence of a light chain variable region set forth in any one of SEQ ID NOs: 20-24; or
   3 CDRs in the amino acid sequence of a heavy chain variable region set forth in SEQ ID NO: 19 and 3 CDRs in the amino acid sequence of a light chain variable region set forth in any one of SEQ ID NOs: 20-24;
(b) 3 CDRs in the amino acid sequence of a heavy chain variable region set forth in SEQ ID NO: 3 and 3 CDRs in the amino acid sequence of a light chain variable region set forth in SEQ ID NO: 4; or a variant with one or more CDRs each having no more than 3 amino acid modifications relative to the 6 CDR regions described above; or
(c) 3 CDRs in the amino acid sequence of a heavy chain variable region set forth in SEQ ID NO: 5 and 3 CDRs in the amino acid sequence of a light chain variable region set forth in SEQ ID NO: 6; or a variant with one or more CDRs each having no more than 3 amino acid modifications relative to the 6 CDR regions described above;
   the amino acid modification is an addition, deletion, or substitution of an amino acid; for example, the amino acid modification is a conservative amino acid substitution.

There are several methods known in the art for determining the CDR sequences of a given antibody molecule. The CDR sequences from the heavy and light chain variable regions of the antibody can be determined according to any method known in the art, including but not limited to the Kabat, Chothia, and IMGT numbering schemes, combinations thereof, or the like.

In some embodiments, the present disclosure provides an isolated antibody or an antigen-binding fragment specifically binding to a Nectin-4 protein, which comprises, according to Kabat numbering:
(a) an HCDR1 set forth in SEQ ID NO: 40 or a variant of the HCDR1 set forth in SEQ ID NO: 40 having no more than 2 amino acid modifications, an HCDR2 set forth in SEQ ID NO: 41 or a variant of the HCDR2 set forth in SEQ ID NO: 41 having no more than 3 amino acid modifications, and an HCDR3 set forth in SEQ ID NO: 42 or a variant of the HCDR3 set forth in SEQ ID NO: 42 having no more than 2 amino acid modifications; an LCDR1 set forth in SEQ ID NO: 43 or a variant of the LCDR1 set forth in SEQ ID NO: 43 having no more than 3 amino acid modifications, an LCDR2 set forth in SEQ ID NO: 44 or a variant of the LCDR2 set forth in SEQ ID NO: 44 having no more than 3 amino acid modifications, and an LCDR3 set forth in SEQ ID NO: 45 or a variant of the LCDR3 set forth in SEQ ID NO: 45 having no more than 2 amino acid modifications;
(b) an HCDR1 set forth in SEQ ID NO: 46 or a variant of the HCDR1 set forth in SEQ ID NO: 46 having no more than 2 amino acid modifications, an HCDR2 set forth in SEQ ID NO: 47 or a variant of the HCDR2 set forth in SEQ ID NO: 47 having no more than 3 amino acid modifications, and an HCDR3 set forth in SEQ ID NO: 48 or a variant of the HCDR3 set forth in SEQ ID NO: 48 having no more than 2 amino acid modifications; an LCDR1 set forth in SEQ ID NO: 49 or a variant of the LCDR1 set forth in SEQ ID NO: 49 having no more than 3 amino acid modifications, an LCDR2 set forth in SEQ ID NO: 50 or a variant of the LCDR2 set forth in SEQ ID NO: 50 having no more than 3 amino acid modifications, and an LCDR3 set forth in SEQ ID NO: 51 or a variant of the LCDR3 set forth in SEQ ID NO: 51 having no more than 2 amino acid modifications; or
(c) an HCDR1 set forth in SEQ ID NO: 52 or a variant of the HCDR1 set forth in SEQ ID NO: 52 having no more than 2 amino acid modifications, an HCDR2 set forth in SEQ ID NO: 53 or a variant of the HCDR2 set forth in SEQ ID NO: 53 having no more than 3 amino acid modifications, and an HCDR3 set forth in SEQ ID NO: 54 or a variant of the HCDR3 set forth in SEQ ID NO: 54 having no more than 2 amino acid modifications; an LCDR1 set forth in SEQ ID NO: 55 or a variant of the LCDR1 set forth in SEQ ID NO: 55 having no more than 3 amino acid modifications, an LCDR2 set forth in SEQ ID NO: 56 or a variant of the LCDR2 set forth in SEQ ID NO: 56 having no more than 3 amino acid modifications, and an LCDR3 set forth in SEQ ID NO: 57 or a variant of the LCDR3 set forth in SEQ ID NO: 57 having no more than 2 amino acid modifications.

Preferably, the isolated antibody or the antigen-binding fragment specifically binding to the Nectin-4 protein comprises, according to Kabat numbering:
(a) an HCDR1 set forth in SEQ ID NO: 40, an HCDR2 set forth in SEQ ID NO: 41, and an HCDR3 set forth in SEQ ID NO: 42; an LCDR1 set forth in SEQ ID NO: 43, an LCDR2 set forth in SEQ ID NO: 44, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 59, an LCDR2 set forth in SEQ ID NO: 61, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 59, an LCDR2 set forth in SEQ ID NO: 44, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 43, an LCDR2 set forth in SEQ ID NO: 62, and an LCDR3 set forth in SEQ ID NO: 45;
   an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 60, an LCDR2 set forth in SEQ ID NO: 62, and an LCDR3 set forth in SEQ ID NO: 45;
(b) an HCDR1 set forth in SEQ ID NO: 46, an HCDR2 set forth in SEQ ID NO: 47, and an HCDR3 set forth in SEQ ID NO: 48; an LCDR1 set forth in SEQ ID NO: 49, an LCDR2 set forth in SEQ ID NO: 50, and an LCDR3 set forth in SEQ ID NO: 51; or
(c) an HCDR1 set forth in SEQ ID NO: 52, an HCDR2 set forth in SEQ ID NO: 53, and an HCDR3 set forth in SEQ ID NO: 54; an LCDR1 set forth in SEQ ID NO: 55, an LCDR2 set forth in SEQ ID NO: 56, and an LCDR3 set forth in SEQ ID NO: 57.

In some embodiments, the isolated anti-Nectin-4 protein antibody of the present disclosure binds to a mammalian Nectin-4 protein, such as a human Nectin-4 protein or a monkey Nectin-4 protein.

In some embodiments, the isolated anti-Nectin-4 protein antibody of the present disclosure has one or more of the following properties:
(a) having an ability to bind to a human Nectin-4 molecule expressed on a cell and a cynomolgus monkey Nectin-4 molecule expressed on a cell, as measured by flow cytometry; and
(b) having an ability to specifically bind only to Nectin-4, with no binding to Nectin family members Nectin1, Nectin2, and Nectin3, as measured by ELISA; and/or
(c) binding to a human Nectin-4 protein with a binding dissociation equilibrium constant K_{D} of less than about 10 nM, such as about 1 nM, about 10⁻¹ nM, about 10⁻² nM, or about 10⁻³ nM; and/or binding to a cynomolgus monkey Nectin-4 protein with a binding dissociation equilibrium constant K_{D} of less than about 100 nM, such as about 10 nM, about 1 nM, about 10⁻¹ nM, or about 10⁻² nM, as measured by biolayer interferometry.

In some embodiments, by specifically binding to a Nectin-4 protein, the isolated anti-Nectin-4 protein antibody of the present disclosure prevents the binding of the Nectin-4 protein to the immune checkpoint molecule TIGIT, thereby functioning to inhibit the signaling pathway of the immune checkpoint molecule TIGIT. In addition, the Nectin-4 protein antibody of the present disclosure also has a direct effect on target cells expressing a Nectin-4 protein. Therefore, the Nectin-4 protein antibody of the present disclosure, without being conjugated to any toxin or anti-tumor agent, is capable of enhancing anti-tumor immunity by reversing the inhibition of the immune system by TIGIT. Therefore, the Nectin-4 protein antibody of the present disclosure can block the interaction between Nectin-4 on cancer cells and TIGIT on immune cells such as T cells and NK cells in cancer immunotherapy, thereby restoring the activity of the immune cells such as T cells and NK cells.

In some embodiments, the isolated anti-Nectin-4 protein antibody or the antigen-binding fragment of the present disclosure comprises a heavy chain variable region and a light chain variable region, wherein
(a) the heavy chain variable region comprises the sequence of SEQ ID NO: 1 or a sequence having at least 90%, 91%, 92%, 93%, 94%, 95%, 96%, 97%, 98%, or 99% identity thereto, and the light chain variable region comprises the sequence of SEQ ID NO: 2 or a sequence having at least 90%, 91%, 92%, 93%, 94%, 95%, 96%, 97%, 98%, or 99% identity thereto;
   for example, a heavy chain variable region set forth in SEQ ID NO: 1 and a light chain variable region set forth in SEQ ID NO: 2;
   a heavy chain variable region set forth in SEQ ID NO: 18 and a light chain variable region set forth in SEQ ID NO: 20;
   a heavy chain variable region set forth in SEQ ID NO: 18 and a light chain variable region set forth in SEQ ID NO: 21;
   a heavy chain variable region set forth in SEQ ID NO: 18 and a light chain variable region set forth in SEQ ID NO: 22;
   a heavy chain variable region set forth in SEQ ID NO: 18 and a light chain variable region set forth in SEQ ID NO: 23;
   a heavy chain variable region set forth in SEQ ID NO: 18 and a light chain variable region set forth in SEQ ID NO: 24;
   a heavy chain variable region set forth in SEQ ID NO: 19 and a light chain variable region set forth in SEQ ID NO: 20;
   a heavy chain variable region set forth in SEQ ID NO: 19 and a light chain variable region set forth in SEQ ID NO: 21;
   a heavy chain variable region set forth in SEQ ID NO: 19 and a light chain variable region set forth in SEQ ID NO: 22;
   a heavy chain variable region set forth in SEQ ID NO: 19 and a light chain variable region set forth in SEQ ID NO: 23;
   a heavy chain variable region set forth in SEQ ID NO: 19 and a light chain variable region set forth in SEQ ID NO: 24;
(b) the heavy chain variable region comprises the sequence of SEQ ID NO: 3 or a sequence having at least 90%, 91%, 92%, 93%, 94%, 95%, 96%, 97%, 98%, or 99% identity thereto, and the light chain variable region comprises the sequence of SEQ ID NO: 4 or a sequence having at least 90%, 91%, 92%, 93%, 94%, 95%, 96%, 97%, 98%, or 99% identity thereto;
   for example, a heavy chain variable region set forth in SEQ ID NO: 3 and a light chain variable region set forth in SEQ ID NO: 4; or
(c) the heavy chain variable region comprises the sequence of SEQ ID NO: 5 or a sequence having at least 90%, 91%, 92%, 93%, 94%, 95%, 96%, 97%, 98%, or 99% identity thereto, and the light chain variable region comprises the sequence of SEQ ID NO: 6 or a sequence having at least 90%, 91%, 92%, 93%, 94%, 95%, 96%, 97%, 98%, or 99% identity thereto;
   for example, a heavy chain variable region set forth in SEQ ID NO: 5 and a light chain variable region set forth in SEQ ID NO: 6.

In some embodiments, the isolated anti-Nectin-4 protein antibody or the antigen-binding fragment of the present disclosure comprises:
(a) SEQ ID NO: 11 or a heavy chain sequence having at least 90%, 91%, 92%, 93%, 94%, 95%, 96%, 97%, 98%, or 99% identity thereto, and SEQ ID NO: 12 or a light chain sequence having at least 90%, 91%, 92%, 93%, 94%, 95%, 96%, 97%, 98%, or 99% identity thereto;
   for example, the heavy chain sequence set forth in SEQ ID NO: 11 and the light chain sequence set forth in SEQ ID NO: 12;
   the heavy chain sequence set forth in SEQ ID NO: 25 and the light chain sequence set forth in SEQ ID NO: 27;
   the heavy chain sequence set forth in SEQ ID NO: 25 and the light chain sequence set forth in SEQ ID NO: 28;
   the heavy chain sequence set forth in SEQ ID NO: 25 and the light chain sequence set forth in SEQ ID NO: 29;
   the heavy chain sequence set forth in SEQ ID NO: 25 and the light chain sequence set forth in SEQ ID NO: 30;
   the heavy chain sequence set forth in SEQ ID NO: 25 and the light chain sequence set forth in SEQ ID NO: 31;
   the heavy chain sequence set forth in SEQ ID NO: 26 and the light chain sequence set forth in SEQ ID NO: 27;
   the heavy chain sequence set forth in SEQ ID NO: 26 and the light chain sequence set forth in SEQ ID NO: 28;
   the heavy chain sequence set forth in SEQ ID NO: 26 and the light chain sequence set forth in SEQ ID NO: 29;
   the heavy chain sequence set forth in SEQ ID NO: 26 and the light chain sequence set forth in SEQ ID NO: 30;
   the heavy chain sequence set forth in SEQ ID NO: 26 and the light chain sequence set forth in SEQ ID NO: 31;
(b) SEQ ID NO: 13 or a heavy chain sequence having at least 90%, 91%, 92%, 93%, 94%, 95%, 96%, 97%, 98%, or 99% identity thereto, and SEQ ID NO: 14 or a light chain sequence having at least 90%, 91%, 92%, 93%, 94%, 95%, 96%, 97%, 98%, or 99% identity thereto; or
(c) SEQ ID NO: 15 or a heavy chain sequence having at least 90%, 91%, 92%, 93%, 94%, 95%, 96%, 97%, 98%, or 99% identity thereto, and SEQ ID NO: 16 or a light chain sequence having at least 90%, 91%, 92%, 93%, 94%, 95%, 96%, 97%, 98%, or 99% identity thereto.

In some embodiments, the isolated anti-Nectin-4 protein antibody of the present disclosure comprises an Fc region from an IgG, e.g., IgG1, IgG2, IgG3, or IgG4. In some embodiments, the Fc region is from IgG1 or IgG4. In some embodiments, the Fc region is from human IgG1 or human IgG4.

In some embodiments, the isolated anti-Nectin-4 protein antibody of the present disclosure comprises a constant region sequence selected from the following: mouse IgG and human IgG, e.g., human IgG1 or human IgG4.

In some embodiments, the isolated anti-Nectin-4 protein antibody of the present disclosure is a chimeric antibody comprising a murine antibody variable region and a human antibody constant region.

In some embodiments, the isolated anti-Nectin-4 protein antibody of the present disclosure is a humanized antibody.

In some embodiments, the amino acid modifications present in the isolated anti-Nectin-4 protein antibody of the present disclosure include amino acid substitutions, insertions, or deletions. Preferably, the amino acid modifications described herein are amino acid substitutions, preferably conservative substitutions. The conservative substitution refers to a substitution of one amino acid with another amino acid of the same class, for example, a substitution of one acidic amino acid with another acidic amino acid, a substitution of one basic amino acid with another basic amino acid, or a substitution of one neutral amino acid with another neutral amino acid. In a preferred embodiment, the amino acid modifications described in the present disclosure can also occur in regions other than CDRs (e.g., in FRs). More preferably, the amino acid modifications described in the present disclosure occur in the Fc regions. In some embodiments, provided is an anti-Nectin-4 protein antibody comprising an Fc domain containing one or more mutations that enhance or diminish the binding of the antibody to an FcRn receptor, e.g., at acidic pH as compared with neutral pH. For example, the present disclosure includes an anti-Nectin-4 protein antibody containing a mutation(s) in the C_{H}2 or C_{H}3 region of the Fc domain, wherein the one or more mutations improve the affinity of the Fc domain for FcRn in an acidic environment (e.g., in an endosome where the pH ranges from about 5.5 to about 6.0). Such mutations can result in an increase in the serum half-life of the antibody when administered to an animal. Non-limiting examples of such Fc modifications include, for example: modifications at positions 250 (e.g., E or Q), 250 and 428 (e.g., L or F), 252 (e.g., L/Y/F/W or T), 254 (e.g., S or T), and 256 (e.g., S/R/Q/E/D or T); modifications at positions 428 and/or 433 (e.g., H/L/R/S/P/Q or K) and/or 434 (e.g., A, W, H, F, or Y [N434A, N434W, N434H, N434F, or N434Y]); modifications at positions 250 and/or 428; or modifications at positions 307 or 308 (e.g., 308F or V308F), and 434. In one embodiment, the modification includes 428L (e.g., M428L) and 434S (e.g., N434S) modifications; 428L, 259I (e.g., V259I), and 308F (e.g., V308F) modifications; 433K (e.g., H433K) and 434 (e.g., 434Y) modifications; 252, 254, and 256 (e.g., 252Y, 254T, and 256E) modifications; 250Q and 428L modifications (e.g., T250Q and M428L); and 307 and/or 308 modifications (e.g., 308F or 308P). In yet another embodiment, the modification includes 265A (e.g., D265A) and/or 297A (e.g., N297A) modifications.

For example, the present disclosure includes an anti-Nectin-4 protein antibody comprising an Fc domain, and the Fc domain contains one pair (group) or more pairs (groups) of mutations selected from the following: 250Q and 248L (e.g., T250Q and M248L); 252Y, 254T, and 256E (e.g., M252Y, S254T, and T256E); 428L and 434S (e.g., M428L and N434S); 257I and 311I (e.g., P257I and Q311I); 257I and 434H (e.g., P257I and N434H); 376V and 434H (e.g., D376V and N434H); 307A, 380A, and 434A (e.g., T307A, E380A, and N434A); and 433K and 434F (e.g., H433K and N434F). Any possible combination of the foregoing Fc domain mutations and other mutations within the antibody variable domains disclosed herein is included within the scope of the present disclosure.

In some embodiments, the isolated anti-Nectin-4 protein antibody provided herein is modified to increase or decrease its glycosylation extent. Addition or deletion of glycosylation sites of the Nectin-4 protein antibody can be conveniently achieved by modifying an amino acid sequence to create or remove one or more glycosylation sites. When the Nectin-4 protein antibody comprises an Fc region, the carbohydrate linked to the Fc region may be altered. In some applications, modifications that remove undesired glycosylation sites may be useful, for example, removing fucose modules to enhance antibody-dependent cellular cytotoxicity (ADCC) (see Shield et al., (2002) JBC277: 26733). In other applications, galactosylation modifications can be made to regulate complement-dependent cytotoxicity (CDC). In certain embodiments, one or more amino acid modifications can be introduced into the Fc region of the Nectin-4 protein antibody provided herein, thereby producing an Fc region variant to enhance, for example, the effectiveness of the isolated anti-Nectin-4 protein antibody of the present disclosure in diagnosing, preventing, and/or treating a disease.

In some embodiments, the isolated anti-Nectin-4 protein antibody or the antigen-binding fragment of the present disclosure can be used for conjugation to a drug to form an antibody-drug conjugate (ADC). Such drugs for conjugation include, but are not limited to, chemotherapeutic agents, immunomodulatory agents (e.g., immunopotentiating agents), oligonucleotides, toxins, radiotoxins, cytokines, apoptotic agents, anti-angiogenic agents, or lymphokines.

In some embodiments, the drug conjugated to the isolated anti-Nectin-4 protein antibody or the antigen-binding fragment of the present disclosure is a cytotoxin.

In some embodiments, the isolated anti-Nectin-4 protein antibody or the antigen-binding fragment of the present disclosure is conjugated to a diagnostic or detectable reagent. Such immunoconjugates can be used to monitor the development, the severity, and/or the prognosis of a disease. Such diagnosis and detection can be accomplished by conjugating the antibody to a detectable substance including, but not limited to, various enzymes, prosthetic groups, fluorescent substances, luminescent substances, and positron-generating metals using various positron emission tomographies, and non-radioactive paramagnetic metal ions.

In some embodiments, the isolated anti-Nectin-4 protein antibody or the antigen-binding fragment of the present disclosure can be used as part of a multispecific antibody for preparing the multispecific antibody.

### III. Multispecific Antibody of the Present Disclosure

Although monoclonal antibodies have been established as anti-tumor therapeutic agents in the past two decades, monoclonal antibodies have a limited ability to mobilize immune cells such as T cells and NK cells at tumor sites and enable immune cells to effectively exert cytotoxic activity. Since multispecific antibodies (e.g., bispecific antibodies) can specifically bind to different antigens, when one antigen is located on a specific immune cell and another antigen is located on a disease cell, the multispecific antibodies (e.g., bispecific antibodies) can redirect the specific immune cell to the disease cell to enhance the killing efficacy of the immune cell against the disease cell. In addition, multispecific antibodies (e.g., bispecific antibodies) can also be designed to act on signal transduction pathways of two or more different mediators simultaneously. Large quantities of imaginative multispecific antibody (e.g., bispecific antibody) formats have been developed through antibody engineering and their applicability in diseases has been studied (Brinkmann U. and Kontermann R. E., The making of bispecific antibodies, Mabs, 2017, 9(2): 182-212).

Multispecific antibodies (e.g., bispecific antibodies) can be classified into many types based on different components and construction methods. For example, based on the substantial bilateral symmetry of multispecific antibody structures, they can be classified into symmetrical antibodies and asymmetric antibodies; based on the presence or absence of IgG Fc region in multispecific antibodies, they can be classified into antibody formats with an Fc region and antibody formats without an Fc region; based on the amount of antigen-binding sites in multispecific antibodies, they can be classified into bivalent, trivalent, or tetravalent antibodies or those of greater valencies, and the like.

The present disclosure provides a multispecific antibody capable of simultaneously binding to a molecule on an immune cell and a Nectin-4 protein on a tumor cell.

In some embodiments, the multispecific antibody of the present disclosure comprises an antibody (e.g., the anti-Nectin-4 protein antibody of the present disclosure) or an antigen-binding fragment specifically binding to a Nectin-4 protein and an antibody or an antigen-binding fragment binding to a co-stimulatory molecule on an immune cell. The co-stimulatory molecule can be represented by the following protein families: TNF receptor proteins, immunoglobulin-like proteins, cytokine receptors, integrins, signaling lymphocytic activation molecules (SLAM proteins), and NK cell receptors. Examples of such molecules include, but are not limited to, CD27, CD28, 4-1BB (CD137), CD86, OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, SLAMF7, NKp80, CD160, B7-H3, and ligands specifically binding to CD83.

In some exemplary embodiments of the present disclosure, the present disclosure provides a bispecific antibody comprising an antibody (e.g., the anti-Nectin-4 protein antibody of the present disclosure) or an antigen-binding fragment specifically binding to a Nectin-4 protein and an antibody or an antigen-binding fragment binding to a co-stimulatory molecule on an immune cell (e.g., an antibody or an antigen-binding fragment binding to a T cell co-stimulatory receptor such as ICOS, 4-1BB, CD28, or CD86).

The isolated anti-Nectin-4 protein antibody or the antigen-binding fragment of the present disclosure contained in the bispecific antibody of the present disclosure comprises at least Nectin-4 protein-binding domains, i.e., at least a heavy chain variable domain and a light chain variable domain specifically binding to a Nectin-4 protein. The antibody or the antigen-binding fragment binding to a co-stimulatory molecule on an immune cell contained in the bispecific antibody of the present disclosure comprises at least domains binding to a co-stimulatory molecule on an immune cell, i.e., at least a heavy chain variable domain and a light chain variable domain specifically binding to a co-stimulatory molecule on an immune cell.

The Nectin-4 protein-binding domains and the domains binding to the co-stimulatory molecule on the immune cell may be linked directly or indirectly to each other to form the bispecific antibody molecule of the present disclosure. Alternatively, the Nectin-4 protein-binding domains and the domains binding to the co-stimulatory molecule on the immune cell may each be linked to a respective multimerization domain. The association of one multimerization domain with another multimerization domain promotes the association of the Nectin-4 protein-binding domains with the domains binding to the co-stimulatory molecule on the immune cell, thereby forming a bispecific antibody molecule. The multimerization domain may be a polypeptide comprising an immunoglobulin C_{H}3 domain, e.g., an Fc moiety of an immunoglobulin (including C_{H}2-C_{H}3 domains), such as an Fc domain of IgG selected from isotypes IgG1, IgG2, IgG3, and IgG4, and any isotype within each isotype group.

The bispecific antibodies of the present disclosure can be prepared using any bispecific antibody format or technique. Specific exemplary bispecific formats that can be used in the present disclosure include, but are not limited to, e.g., scFv-based or diabody bispecific formats, and bispecific formats of IgG-scFv fusion, dual variable domain (OVO)-Ig, quadroma, knobs-into-holes, common light chain (e.g., common light chains having knobs-into-holes, etc.), CrossMab, CrossFab, (SEEO)body, leucine zipper, Ouobody, IgG1/lgG2, dual-action Fab (OAF)-lgG, and Mab² (see, e.g., Klein et al., 2012, mAbs, 4:6, 1-11, and references cited therein).

In some embodiments, the bispecific antibody of the present disclosure comprises, from the amino-terminus to the carboxyl-terminus:
(a) a first moiety, which is the Nectin-4 protein antibody or the antigen-binding fragment specifically binding to the Nectin-4 protein of the present disclosure, and
(b) a second moiety, which is an anti-4-1BB scFv,
   for example, the anti-4-1BB scFv comprises 3 CDRs in the amino acid sequence of a heavy chain variable region set forth in SEQ ID NO: 32 and 3 CDRs in the amino acid sequence of a light chain variable region set forth in SEQ ID NO: 33; or a variant with one or more CDRs each having no more than 3 amino acid modifications relative to the 6 CDR regions described above.

In some embodiments, the bispecific antibody of the present disclosure comprises, from the amino-terminus to the carboxyl-terminus:
(a) a first moiety, which is the Nectin-4 protein antibody or the antigen-binding fragment specifically binding to the Nectin-4 protein of the present disclosure, and
(b) a second moiety, which is an anti-4-1BB scFv comprising, according to Kabat numbering, an HCDR1 set forth in SEQ ID NO: 63, an HCDR2 set forth in SEQ ID NO: 64, and an HCDR3 set forth in SEQ ID NO: 65; an LCDR1 set forth in SEQ ID NO: 66, an LCDR2 set forth in SEQ ID NO: 67, and an LCDR3 set forth in SEQ ID NO: 68.

In some embodiments, the bispecific antibody of the present disclosure comprises, from the amino-terminus to the carboxyl-terminus:
(a) a first moiety, which is the Nectin-4 protein antibody or the antigen-binding fragment specifically binding to the Nectin-4 protein of the present disclosure, and
(b) a second moiety, which is an anti-4-1BB scFv comprising a heavy chain variable region set forth in SEQ ID NO: 32 or a sequence having at least 90%, 91%, 92%, 93%, 94%, 95%, 96%, 97%, 98%, or 99% identity thereto, and a light chain variable region set forth in SEQ ID NO: 33 or a sequence having at least 90%, 91%, 92%, 93%, 94%, 95%, 96%, 97%, 98%, or 99% identity thereto;
wherein the carboxyl-terminus of the heavy chain of the first moiety and the amino-terminus of the second moiety are linked covalently.

In some embodiments, the bispecific antibody of the present disclosure comprises two heavy chains and two light chains, wherein each heavy chain comprises, from the amino-terminus to the carboxyl-terminus, the heavy chain of the first moiety, a linker peptide, and the second moiety, and each light chain is the light chain of the first moiety; preferably, each heavy chain comprises the amino acid sequence set forth in SEQ ID NO: 35 or a sequence having at least 80%, 85%, 90%, 91%, 92%, 93%, 94%, 95%, 96%, 97%, 98%, 99%, or higher identity thereto; and each light chain comprises the amino acid sequence set forth in SEQ ID NO: 12 or a sequence having at least 80%, 85%, 90%, 91%, 92%, 93%, 94%, 95%, 96%, 97%, 98%, 99%, or higher identity thereto;

each heavy chain comprises the amino acid sequence set forth in SEQ ID NO: 36 or a sequence having at least 80%, 85%, 90%, 91%, 92%, 93%, 94%, 95%, 96%, 97%, 98%, 99%, or higher identity thereto; and each light chain comprises the amino acid sequence set forth in SEQ ID NO: 16 or a sequence having at least 80%, 85%, 90%, 91%, 92%, 93%, 94%, 95%, 96%, 97%, 98%, 99%, or higher identity thereto; or

each heavy chain comprises the amino acid sequence set forth in SEQ ID NO: 37 or a sequence having at least 80%, 85%, 90%, 91%, 92%, 93%, 94%, 95%, 96%, 97%, 98%, 99%, or higher identity thereto; and each light chain comprises the amino acid sequence set forth in SEQ ID NO: 31 or a sequence having at least 80%, 85%, 90%, 91%, 92%, 93%, 94%, 95%, 96%, 97%, 98%, 99%, or higher identity thereto.

The type of the linker peptide is not particularly limited. In an embodiment, the linker peptide is a peptide having an amino acid sequence of 1 to 100, particularly 1 to 50, and more particularly 1 to 20 amino acids in length. In some embodiments, the linker peptide is (GxS)n or (GxS)nGm, wherein G = glycine, S = serine, x = any integer of 1 to 4, n = any integer of 1 to 7, and m = any integer of 0 to 3. In one specific embodiment, the linker peptide is (G₄S)₃ (SEQ ID NO: 69).

In some embodiments, the anti-Nectin-4×4-1BB bispecific antibody of the present disclosure has one or more of the following properties:
(a) binding to a human Nectin-4 protein with a binding dissociation equilibrium constant K_{D} of less than about 10 nM, such as about 1 nM, about 10⁻¹ nM, about 10⁻² nM, or about 10⁻³ nM, and binding to a human 4-1BB protein with a binding dissociation equilibrium constant K_{D} of less than about 1000 nM, such as about 100 nM, about 10 nM, about 1 nM, or about 10⁻¹ nM; or binding to a cynomolgus monkey Nectin-4 protein with a binding dissociation equilibrium constant K_{D} of less than about 100 nM, such as about 10 nM, about 1 nM, about 10⁻¹ nM, or about 10⁻² nM, and binding to a cynomolgus monkey 4-1BB protein with a binding dissociation equilibrium constant K_{D} of less than about 150 nM, such as about 15 nM, about 1.5 nM, about 0.15 nM, or about 0.015 nM, as measured by biolayer interferometry;
(b) the bispecific antibody being able to activate a human 4-1BB signaling pathway only in the presence of a Nectin-4 protein; the bispecific antibody being not able to activate the human 4-1BB signaling pathway in the absence of the Nectin 4 protein, as measured in an NF-κB-Luc/4-1BB reporter gene cell line; and/or
(c) having *in vivo* anti-tumor efficacy, and the bispecific antibody being safe to test animals.

In the bispecific antibody of the present disclosure, the Fc domain may comprise one or more amino acid modifications (e.g., insertions, deletions, or substitutions), as compared with a wild-type or naturally occurring form of the Fc domain.

In some embodiments, the present disclosure provides a bispecific antibody comprising an Fc domain containing one or more mutations that enhance or diminish the binding of the bispecific antibody to an FcRn receptor, e.g., at acidic pH as compared with neutral pH. For example, the present disclosure includes a bispecific antibody containing a mutation(s) in the C_{H}2 or C_{H}3 region of the Fc domain, wherein the one or more mutations improve the affinity of the Fc domain for FcRn in an acidic environment (e.g., in an endosome where the pH ranges from about 5.5 to about 6.0). Such mutations can result in an increase in the serum half-life of the antibody when administered to an animal. Non-limiting examples of such Fc modifications include, for example: modifications at positions 250 (e.g., E or Q), 250 and 428 (e.g., L or F), 252 (e.g., L/Y/F/W or T), 254 (e.g., S or T), and 256 (e.g., S/R/Q/E/D or T); modifications at positions 428 and/or 433 (e.g., H/L/R/S/P/Q or K) and/or 434 (e.g., A, W, H, F, or Y [N434A, N434W, N434H, N434F, or N434Y]); modifications at positions 250 and/or 428; or modifications at positions 307 or 308 (e.g., 308F or V308F), and 434. In one embodiment, the modification includes 428L (e.g., M428L) and 434S (e.g., N434S) modifications; 428L, 259I (e.g., V259I), and 308F (e.g., V308F) modifications; 433K (e.g., H433K) and 434 (e.g., 434Y) modifications; 252, 254, and 256 (e.g., 252Y, 254T, and 256E) modifications; 250Q and 428L modifications (e.g., T250Q and M428L); and 307 and/or 308 modifications (e.g., 308F or 308P). In yet another embodiment, the modification includes 265A (e.g., D265A) and/or 297A (e.g., N297A) modifications.

For example, the present disclosure includes a bispecific antibody comprising an Fc domain, and the Fc domain contains one pair (group) or more pairs (groups) of mutations selected from the following: 250Q and 248L (e.g., T250Q and M248L); 252Y, 254T, and 256E (e.g., M252Y, S254T, and T256E); 428L and 434S (e.g., M428L and N434S); 257I and 311I (e.g., P257I and Q311I); 257I and 434H (e.g., P257I and N434H); 376V and 434H (e.g., D376V and N434H); 307A, 380A, and 434A (e.g., T307A, E380A, and N434A); and 433K and 434F (e.g., H433K and N434F). Any possible combination of the foregoing Fc domain mutations and other mutations within the antibody variable domains disclosed herein is included within the scope of the present disclosure.

In some embodiments, the bispecific antibody provided herein is modified to increase or decrease its glycosylation extent. Addition or deletion of glycosylation sites of the bispecific antibody can be conveniently achieved by modifying an amino acid sequence to create or remove one or more glycosylation sites. When the bispecific antibody comprises an Fc region, the carbohydrate linked to the Fc region may be altered. In some applications, modifications that remove undesired glycosylation sites may be useful, for example, removing fucose modules to enhance antibody-dependent cellular cytotoxicity (ADCC) (see Shield et al., (2002) JBC277: 26733). In other applications, galactosylation modifications can be made to regulate complement-dependent cytotoxicity (CDC).

### IV. Nucleic Acid of the Present Disclosure and Host Cell Comprising Same

In one aspect, the present disclosure provides a nucleic acid encoding any of the above Nectin-4 protein antibodies or the antigen-binding fragments thereof, or any one of the chains thereof. In another aspect, the present disclosure provides a nucleic acid encoding any of the above multispecific antibodies of the present disclosure or any one of the chains thereof.

In one embodiment, provided is a vector comprising the above nucleic acids. In one embodiment, the vector is an expression vector. In one embodiment, provided is a host cell comprising the nucleic acid or the vector. In one embodiment, the host cell is eukaryotic. In another embodiment, the host cell is selected from a yeast cell, a mammalian cell (e.g., a CHO cell or an HEK 293 cell), and other cells suitable for preparing an antibody or an antigen-binding fragment thereof. In another embodiment, the host cell is prokaryotic.

The present disclosure also encompasses nucleic acids hybridized with the above nucleic acids under strict conditions or nucleic acids encoding polypeptide sequences having one or more amino acid substitutions (e.g., conservative substitutions), deletions, or insertions, as compared with the above nucleic acids.

In one embodiment, provided are one or more vectors comprising the above nucleic acids. In one embodiment, the vector is an expression vector, e.g., a eukaryotic expression vector. The vector includes, but is not limited to, a virus, a plasmid, a cosmid, a λ phage, or a yeast artificial chromosome (YAC).

Once the expression vector or DNA sequence for expression has been prepared, the expression vector can be transfected or introduced into a suitable host cell. Various techniques may be used for achieving this purpose, for example, protoplast fusion, calcium phosphate precipitation, electroporation, retroviral transduction, viral transfection, biolistics, lipid-based transfection, or other conventional techniques. In the case of protoplast fusion, cells are cultured in a culture medium and screened for appropriate activity. Methods and conditions for culturing the resulting transfected cells and for recovering the resulting antibody molecules are known to those skilled in the art and may be modified or optimized according to the particular expression vectors and the particular mammalian host cells used, based on this description and methods known in the art.

In addition, one or more markers enabling the selection of transfected host cells may be introduced to select the cells in which DNA has been stably incorporated into the chromosome(s) thereof. The marker may, for example, provide prototrophy, biocidal resistance (e.g., antibiotics), or resistance to heavy metals (e.g., copper), etc., to an auxotrophic host. The selectable marker gene may be linked directly to a DNA sequence to be expressed or introduced into the same cell by co-transformation. Additional elements may also be required for optimal synthesis of mRNA. These elements may include splicing signals, transcriptional promoters, enhancers, and termination signals.

In one embodiment, provided is a host cell comprising the polynucleotide of the present disclosure. In some embodiments, provided is a host cell comprising the expression vector of the present disclosure. In some embodiments, the host cell is selected from a yeast cell, a mammalian cell, and other cells suitable for preparing an antibody. Suitable host cells include prokaryotic microorganisms, such as *Escherichia coli.* The host cell may also be a eukaryotic microorganism such as a filamentous fungus or yeast, or various eukaryotic cells such as insect cells, etc. Vertebrate cells may also be used as hosts. For example, a mammalian cell line engineered to be suitable for suspension growth may be used. Examples of useful mammalian host cell lines include the SV40-transformed monkey kidney CV1 line (COS-7), human embryonic kidney line (HEK 293 or 293F cells), baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (Hep G2), Chinese hamster ovary cells (CHO cells), CHOS cells, NSO cells, and myeloma cell lines such as Y0, NS0, P3X63, Sp2/0, and the like. For reviews of mammalian host cell lines suitable for protein production, see, e.g., Yazaki and Wu, Methods in Molecular Biology, Vol. 248 (edited by B. K. C. Lo, Humana Press, Totowa, NJ), pages 255-268 (2003). In one preferred embodiment, the host cell is an HEK 293 cell or a CHO cell, e.g., an HEK 293-6E cell.

### V. Production and Purification of Isolated Anti-Nectin-4 Protein Antibody and Multispecific Antibody Comprising Same of the Present Disclosure

In one embodiment, the present disclosure provides a method for preparing an isolated anti-Nectin-4 protein antibody or a multispecific antibody comprising same, wherein the method comprises culturing a host cell comprising a nucleic acid encoding the isolated anti-Nectin-4 protein antibody or the multispecific antibody or comprising an expression vector comprising the nucleic acid under conditions suitable for expressing the nucleic acid encoding the Nectin-4 protein antibody or suitable for expressing the nucleic acid encoding the multispecific antibody, and optionally isolating the Nectin-4 protein antibody or the multispecific antibody. In a certain embodiment, the method further comprises recovering the Nectin-4 protein antibody or the multispecific antibody from the host cell (or a host cell culture medium).

For recombinant production of the isolated anti-Nectin-4 protein antibody or the multispecific antibody of the present disclosure, a nucleic acid encoding the Nectin-4 protein antibody or the multispecific antibody of the present disclosure is isolated and then inserted into a vector for further cloning and/or expression in a host cell. Such nucleic acids can be easily isolated and sequenced by using conventional procedures, for example, by using an oligonucleotide probe capable of specifically binding to the nucleic acid encoding the Nectin-4 protein antibody or the multispecific antibody of the present disclosure.

The anti-Nectin-4 protein antibody or the multispecific antibody of the present disclosure prepared as described herein may be purified by known prior art such as high-performance liquid chromatography, ion exchange chromatography, gel electrophoresis, affinity chromatography, and size exclusion chromatography. The actual conditions used for purifying a particular protein also depend on factors including net charge, hydrophobicity, hydrophilicity, and the like, and such conditions would be apparent to those skilled in the art. The purity of the Nectin-4 protein antibody or the multispecific antibody of the present disclosure may be determined by any one of a variety of well-known analytical methods including size exclusion chromatography, gel electrophoresis, high-performance liquid chromatography, and the like.

### VI. Activity Assay on Isolated Anti-Nectin-4 Protein Antibody or Antigen-Binding Fragment Thereof or Multispecific Antibody Comprising Same of the Present Disclosure

The Nectin-4 protein antibody or the multispecific antibody provided herein may be identified, screened, or characterized for physical/chemical properties and/or biological activities thereof through a variety of assays known in the art.

In one aspect, the Nectin-4 protein antibody or the multispecific antibody of the present disclosure is tested for the antigen-binding activity thereof through, for example, known methods such as ELISA. The binding to each antigen targeted by the Nectin-4 protein and/or the multispecific antibody may be assayed by using methods known in the art, and exemplary methods are disclosed herein. In some embodiments, SPR or biolayer interferometry is used to assay the binding of the Nectin-4 protein antibody or the multispecific antibody of the present disclosure to the Nectin-4 protein.

The present disclosure further provides an assay for identifying a multispecific antibody having biological activity. The biological activity may include, for example, measuring activation of a 4-1BB signaling pathway by an anti-Nectin-4×4-1BB bispecific antibody using an HEK293/NF-κB-Luc/4-1BB reporter gene cell line.

### VII. Pharmaceutical Composition and Pharmaceutical Formulation

In some embodiments, the present disclosure provides a composition comprising any of the isolated anti-Nectin-4 protein antibodies or the multispecific antibodies described herein, and preferably, the composition is a pharmaceutical composition. In one embodiment, the composition further comprises a pharmaceutical supplementary material. In one embodiment, the composition (e.g., the pharmaceutical composition) comprises the Nectin-4 protein antibody or the multispecific antibody of the present disclosure, and a combination of one or more additional therapeutic agents (e.g., a chemotherapeutic drug, a tumor vaccine, an antibody binding to a specific antigen on a tumor cell, or a tumor cell-depleting antibody).

In some embodiments, the pharmaceutical composition or the pharmaceutical formulation of the present disclosure comprises a suitable pharmaceutical supplementary material, such as a pharmaceutical carrier and a pharmaceutical excipient known in the art, including buffers.

As used herein, "pharmaceutical carrier" includes any and all solvents, dispersion media, isotonic agents, absorption delaying agents, and the like that are physiologically compatible. The pharmaceutical carrier suitable for use in the present disclosure may be a sterile liquid, such as water and oil, including those of petroleum, animal, plant, or synthetic origin, such as peanut oil, soybean oil, mineral oil, or sesame oil. The isolated anti-Nectin-4 protein antibody or the multispecific antibody of the present disclosure with a desired purity may be mixed with one or more optional pharmaceutical supplementary materials (Remington's Pharmaceutical Sciences, 16th Ed. edited by Osol, A. (1980)) to prepare the pharmaceutical formulation comprising the isolated anti-Nectin-4 protein antibody or the multispecific antibody described herein, preferably in the form of a lyophilized formulation or an aqueous solution.

The pharmaceutical composition or the formulation of the present disclosure may further comprise more than one active ingredient required by a particular indication treated, preferably those having complementarity activity without adversely affecting one another. When used in the treatment of cancers, such active ingredients include, but are not limited to, anti-cancer agents and chemotherapeutic agents; when used in the treatment of infectious diseases, such active ingredients include, but are not limited to, antiviral agents and antibiotics. The active ingredients are properly present in combination in amounts effective for the intended use.

A sustained-release formulation may be prepared. Suitable examples of the sustained-release formulation include a semipermeable matrix of a solid hydrophobic polymer comprising the Nectin-4 protein antibody or the multispecific antibody of the present disclosure, and the matrix is in the form of a shaped article, such as a film or a microcapsule.

### VIII. Combination Product or Kit

In some embodiments, the present disclosure further provides a combination product, comprising at least one of the isolated anti-Nectin-4 protein antibodies or the multispecific antibodies of the present disclosure, or further comprising one or more additional anti-tumor agents.

In some embodiments, in the combination product, two or more ingredients may be administered to a subject in combination sequentially, separately, or simultaneously.

In some embodiments, the present disclosure further provides a kit comprising the isolated anti-Nectin-4 protein antibody, the multispecific antibody, the pharmaceutical composition, or the combination product of the present disclosure, and optionally a package insert directing administration.

In some embodiments, the present disclosure further provides a pharmaceutical product comprising the isolated anti-Nectin-4 protein antibody, the multispecific antibody, the pharmaceutical composition, or the combination product of the present disclosure, and optionally, the pharmaceutical product further comprises a package insert directing administration.

### IX. Use of Isolated Anti-Nectin-4 Protein Antibody or Multispecific Antibody Comprising Same of the Present Disclosure

The isolated anti-nectin-4 protein antibody or the multispecific antibody comprising same disclosed herein has *in vitro* and *in vivo* diagnostic use and therapeutic and prophylactic use. For example, these molecules may be administered to *in vitro* or *ex vivo* cultured cells or administered to a subject, e.g., a human subject, to treat, prevent, and/or diagnose a disease associated with a Nectin-4 protein antigen, e.g., a cancer.

In one aspect, the present disclosure provides a diagnostic method for detecting the presence of a relevant Nectin-4 protein antigen in a biological sample, such as serum, semen, urine, or a tissue biopsy sample (e.g., from a hyperproliferative or cancerous lesion), *in vitro* or *in vivo.* The diagnostic method comprises: (i) contacting a sample (and optionally, a control sample) with the Nectin-4 protein antibody or the multispecific antibody described herein or administering to a subject the Nectin-4 protein antibody or the multispecific antibody under conditions enabling interactions, and (ii) detecting the formation of a complex of the Nectin-4 protein antibody or the multispecific antibody with the sample (and optionally, the control sample). The formation of the complex indicates the presence of the relevant antigen and may show the suitability for or requirements of the treatment and/or prevention described herein.

In some embodiments, the relevant antigen is detected prior to treatment, e.g., prior to an initial treatment or prior to a certain treatment after a treatment interval. Detection methods that can be used include immunohistochemistry, immunocytochemistry, FACS, ELISA assays, PCR techniques (e.g., RT-PCR), or *in vivo* imaging techniques. Generally, the Nectin-4 protein antibody or the multispecific antibody used in the *in vivo* and *in vitro* detection methods is directly or indirectly labeled with a detectable substance to facilitate the detection of bound or unbound conjugates. Suitable detectable substances include various biologically active enzymes, prosthetic groups, fluorescent substances, luminescent substances, paramagnetic (e.g., nuclear magnetic resonance active) substances, and radioactive substances.

In some embodiments, the level and/or distribution of the relevant antigen are determined *in vivo.* For example, the Nectin-4 protein antibody or multispecific antibody of the present disclosure labeled with a detectable substance is detected in a non-invasive manner (e.g., by using appropriate imaging techniques such as positron emission tomography (PET) scanning). In one embodiment, for example, the level and/or distribution of the relevant antigen are assayed *in vivo* by detecting the Nectin-4 protein antibody or the multispecific antibody of the present disclosure that is detectably labeled with a PET reagent (e.g., ¹⁸F-fluorodeoxyglucose (FDG)).

In one embodiment, the present disclosure provides a diagnostic kit comprising the Nectin-4 protein antibody or the multispecific antibody described herein and an instruction for use.

In another aspect, the present disclosure relates to use of the Nectin-4 protein antibody or the multispecific antibody of the present disclosure *in vivo* in treating or preventing a cancer, and modulating an immune response in a subject, thereby inhibiting or reducing the occurrence or recurrence of relevant diseases such as a cancer. The Nectin-4 protein antibody or the multispecific antibody of the present disclosure may be used alone. Alternatively, the Nectin-4 protein antibody or the multispecific antibody may be administered in combination with an additional cancer therapeutic/prophylactic agent. When the Nectin-4 protein antibody or the multispecific antibody of the present disclosure is administered in combination with one or more additional drugs, the combination may be administered in any order or simultaneously.

Thus, in one embodiment, the present disclosure provides a method for treating or preventing a cancer, and modulating an immune response in a subject, which comprises administering to the subject a therapeutically effective amount of the Nectin-4 protein antibody or the multispecific antibody described herein. In another embodiment, the present disclosure provides a method for preventing the occurrence or recurrence of a cancer in a subject, which comprises administering to the subject a prophylactically effective amount of the Nectin-4 protein antibody or the multispecific antibody described herein.

In some embodiments, the cancer treated and/or prevented with the Nectin-4 protein antibody or the multispecific antibody includes, but is not limited to, a solid tumor and a hematological cancer (e.g., leukemia, lymphoma, or myeloma, such as multiple myeloma) expressing a Nectin-4 protein. In some embodiments, the cancer treated and/or prevented with the Nectin-4 protein antibody or the multispecific antibody includes, but is not limited to, a carcinoma *in situ* or a metastatic cancer expressing a Nectin-4 protein.

In one embodiment, the cancer is a solid tumor expressing a Nectin-4 protein. Examples of solid tumors include malignant tumors, e.g., sarcomas and cancers of various organ systems, such as those cancers invading the esophagus, lung, breast, ovary, lymph, gastrointestinal tract (e.g., colon), anus, genital and genitourinary tract (e.g., kidney, bladder epithelium, bladder cells, and prostate), pharynx, CNS (e.g., brain, neurological or glial cells), head and neck, skin (e.g., melanoma), nasopharynx (e.g., differentiated or undifferentiated metastatic or locally recurrent nasopharyngeal carcinoma), and pancreas, as well as adenocarcinomas, including malignant tumors. The cancer may be at an early, intermediate, or advanced stage, or may be a metastatic cancer.

In some embodiments, the cancer is selected from colon cancer, rectal cancer, colorectal cancer, esophageal cancer, skin cancer, urothelial carcinoma, ovarian cancer, pancreatic cancer, bladder cancer, non-Hodgkin's lymphoma, Hodgkin's lymphoma, acute lymphocytic leukemia, multiple myeloma, breast cancer, gastric cancer, hepatocellular carcinoma, non-small cell lung cancer, small cell lung cancer, melanoma, glioblastoma, renal cell carcinoma, and prostate cancer.

The following examples are described to assist in understanding the present disclosure. The examples are not intended to be and shall not be interpreted in any way as limiting the claimed scope of the present disclosure.

### Examples

### Example 1. Production of Anti-Human Nectin-4 Antibodies

The hybridoma of the anti-human Nectin-4 antibody of the present disclosure may be prepared by the method described below. However, the method for preparing the anti-human Nectin-4 antibody of the present disclosure is not limited to this method, and the antibody may also be prepared by other methods known in the art.

### 1.1 Animal immunization

The human Nectin-4-his protein (ACRO Biosystems, Cat. No. NE4-H52H3) was used as an immunogen to immunize 5 BALB/c mice. Immune injection was performed on the BALB/c mice once every two weeks for a total of four times (Table 1). Blood was separately collected intravenously from the mice on day 7 after the second immunization and on day 7 after the third immunization. The titer of the antibody in the serum of the immunized animals was determined by ELISA using the human Nectin-4-his protein. After a sufficient increase in the antibody titer was observed, the last booster immunization was performed. Finally, two immunized mice were selected for obtaining antibody-producing cells. Four days after the last booster immunization, spleens of the immunized mice were taken, and lymphocytes isolated from the spleens were fused with myeloma cells to produce hybridomas.

**Table 1. Immunization procedures for BALB/c mice**

| Procedure | Immunization route | Dose |
|---|---|---|
| Primary immunization | Subcutaneous injection | 50 µg protein/mouse |
| First booster immunization | Subcutaneous injection | 25 µg protein/mouse |
| Second booster immunization | Subcutaneous injection | 25 µg protein/mouse |
| Last booster immunization | Subcutaneous injection | 25 µg protein/mouse |

### 1.2 Screening of target hybridomas

Cell culture supernatants of 4800 hybridomas from Example 1.1 were subjected to primary screening by an ELISA assay using the human Nectin-4-his protein. After the ELISA assay, 198 positive supernatants were selected to detect an MCF-7 cell line (a human breast cancer cell line) by flow cytometry. The hybridoma clones corresponding to the positive supernatants binding to MCF-7 cells were confirmed by the next round of ELISA assay. Finally, 21 hybridoma clones were selected to enter the next subcloning stage.

### 1.3 Subcloning of parental hybridomas

The screened hybridoma cell lines (parental clones) were subcloned to ensure monoclonality. Subcloning was performed by reseeding the parental clones using a single step cloning system. The 21 subclones from Example 1.2 were transferred to a 96-well cell culture plate. The subclones were screened by an ELISA method, and human Nectin-4 (ACRO, Cat. No. NE4-H52H3) and cynomolgus monkey Nectin-4 (KACTUS, Cat. No. NEC-CM104) were used as antigens for screening to obtain hybridomas producing antibodies binding to human Nectin-4 and cynomolgus monkey Nectin-4. The resulting antibodies were designated as: murine antibody 23-H4F3, murine antibody 6-B9B6, and murine antibody 32-A12C7.

### 1.4 Sequencing of anti-Nectin-4 antibodies produced by hybridomas

Antibody sequencing was completed by GenScript NGS. The amino acid sequences of the heavy chain variable regions (VHs) and light chain variable regions (VLs) are shown in Table 2.

**Table 2. Amino acid sequences of heavy chain variable regions and light chain variable regions of anti-Nectin-4 antibodies**

| Antibody name | VH | VL |
|---|---|---|
| Murine antibody 23-H4F3 | SEQ ID NO: 1 | SEQ ID NO: 2 |
| Murine antibody 6-B9B6 | SEQ ID NO: 3 | SEQ ID NO: 4 |
| Murine antibody 32-A12C7 | SEQ ID NO: 5 | SEQ ID NO: 6 |

### Example 2. Characterization of Anti-Nectin-4 Chimeric Antibodies

### 2.1 Synthesis and expression of anti-Nectin-4 chimeric antibodies

The DNA sequences of the murine antibodies in Table 2 were subjected to codon optimization and gene synthesis by General Biology System (Anhui) Co., Ltd. The genes encoding the VH regions of the antibodies were separately inserted into an expression vector pcDNA3.1(+) comprising a gene encoding a human IgG1 heavy chain constant region (SEQ ID NO: 7) to obtain plasmids encoding the heavy chains of the anti-Nectin-4 antibodies. The genes encoding the VL regions of the antibodies were separately inserted into an expression vector pcDNA3.1(+) comprising a gene encoding a human κ light chain constant region (SEQ ID NO: 8) to obtain plasmids encoding the light chains of the anti-Nectin-4 antibodies.

The plasmids encoding the heavy chains and the light chains of the anti-Nectin-4 antibodies were co-transfected into ExpiCHO-S cells to express the murine chimeric antibodies shown in Table 3. Enfortumab, a fully human monoclonal antibody targeting Nectin-4 (heavy chain amino acid sequence: SEQ ID NO: 9; light chain amino acid sequence: SEQ ID NO: 10; with the sequences derived from PCT Publication No. WO2021030240A1), was used as the control antibody. Similarly, the control antibody enfortumab was expressed. The antibodies were purified accordingly.

Specifically, according to the manufacturer's product instructions, the plasmids encoding the heavy chains and the light chains of the anti-Nectin-4 antibodies shown in Table 3 were co-transfected into ExpiCHO-S cells using an ExpiCHO^{™} expression system (ThermoFisher, Cat#: A29133) to express the anti-Nectin-4 chimeric antibodies, and similarly to express the control antibody enfortumab. The cells were cultured for 10-12 days after transfection. When the cell viability decreased to 60% to 70%, the supernatants were collected, and the antibodies expressed and secreted in the supernatants were purified using a MabSelect Sure protein A affinity chromatography system (GE healthcare). The purified antibodies were concentrated, and subjected to sterile filtration. The purities of the antibody proteins were detected by SDS-PAGE and size exclusion chromatography. The results indicate that the purities of the antibodies were greater than 90%, and the antibodies could be used in the next step.

**Table 3. Chimeric antibody names and combinations of the corresponding heavy chains and light chains**

| Name of chimeric antibody | Heavy chain | Light chain |
|---|---|---|
| Antibody 23-H4F3 | Mouse 23-H4F3 VH-human IgG1 CH (SEQ ID NO: 11) | Mouse 23-H4F3 VL-human κ CL (SEQ ID NO: 12) |
| Antibody 6-B9B6 | Mouse 6-B9B6 VH-human IgG1 CH (SEQ ID NO: 13) | Mouse 6-B9B6 VL-human κ CL (SEQ ID NO: 14) |
| Antibody 32-A12C7 | Mouse A12C7 VH-human IgG1 CH (SEQ ID NO: 15) | Mouse A12C7 VL-human κ CL (SEQ ID NO: 16) |

### 2.2 Binding of anti-Nectin-4 chimeric antibodies to HT-1376 or cynomolgus monkey Nectin-4 engineered cells

Whether the anti-Nectin-4 chimeric antibodies in the present disclosure were able to bind to the human Nectin-4 protein expressed by tumor cells (e.g., human bladder cancer cell line HT-1376) or engineered cells expressing the cynomolgus monkey Nectin-4 protein was determined by a cell binding experiment.

The sequence (SEQ ID NO: 17) encoding the extracellular and transmembrane regions of cynomolgus monkey Nectin-4 (XP_005541277.1) was cloned into a PiggyBac Dual promoter (SBI, Cat. No. PB513-B1) expression vector, and the resulting vector was transfected into CHO-K1 cells (ATCC, Cat. No. CCL-61^{™}) by electroporation. The cells were then screened with 8 µg/mL puromycin (Gibco, Cat. No. A1113802) to obtain CHO-K1 cells highly expressing cynomolgus monkey Nectin-4 (hereinafter also referred to as CHO-K1/cynoNectin4).

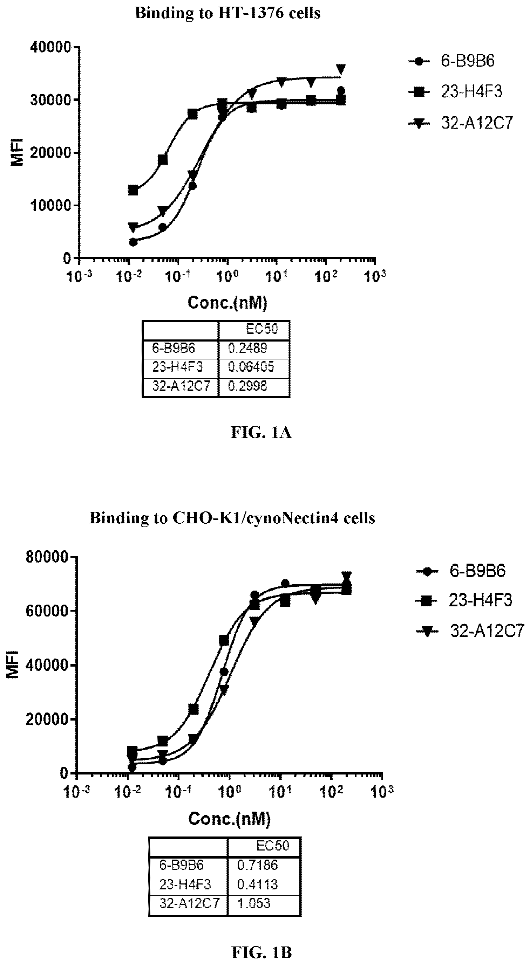

Adherent HT-1376 (Nanjing Cobioer Biosciences Co., Ltd., Cat. No. CBP60310) and CHO-K1/cynoNectin4 cell line were separately subjected to enzymatic digestion to obtain single-cell suspensions of the two types of cells. The suspensions were centrifuged at 300× g at room temperature for 4 min, and then the culture medium was discarded. The resulting cell pellets were washed once with PBS. The cells were resuspended in the murine chimeric anti-Nectin-4 antibodies obtained in Example 2.1 that were serially diluted (at an initial concentration of 200 nM, 4-fold gradient dilution, 8 concentrations in total) and incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat#: 109-116-098) diluted in a 1:200 ratio was added. The cells were incubated in the dark at 4 °C for 30 min. The cells were washed twice with PBS and then resuspended, and finally, two-channel fluorescence signals were detected using a flow cytometer. The results are shown in FIG. 1A and FIG. 1B.

FIG. 1A shows that the murine chimeric anti-Nectin-4 antibodies (i.e., antibody 23-H4F3, antibody 6-B9B6, and antibody 32-A12C7) were all able to bind to the HT-1376 cells expressing human Nectin-4, with the EC50 of the antibody 23-H4F3 being 0.06405 nM, the EC50 of the antibody 6-B9B6 being 0.2489 nM, and the EC50 of the antibody 32-A12C7 being 0.2998 nM. FIG. 1B shows that the murine chimeric anti-Nectin-4 antibodies (i.e., antibody 23-H4F3, antibody 6-B9B6, and antibody 32-A12C7) were also all able to bind to the cells expressing the cynomolgus monkey Nectin-4 protein, with the EC50 of the antibody 23-H4F3 being 0.4113 nM, the EC50 of the antibody 6-B9B6 being 0.7186 nM, and the EC50 of the antibody 32-A12C7 being 1.053 nM. The binding ability of the chimeric antibody 23-H4F3 to each cell was superior to that of antibody 6-B9B6 and antibody 32-A12C7.

### 2.3 Binding of anti-Nectin-4 antibodies to Nectin family

The binding properties of the anti-Nectin-4 antibodies obtained in Example 2.1 to Nectin family members were detected by an ELISA method.

The human Nectin-1 protein (ACRO Biosystems, Cat. No. PV1-H5223), human Nectin-2 protein (Sino Biological, Cat. No. 10005-H08H), human Nectin-3 protein (Sino Biological, Cat. No. 10852-H08H), and human Nectin-4 protein (Sino Biological, Cat. No. HPLC-19771-H08H) were separately immobilized on a 96-well plate by incubation at 4 °C overnight. The 96-well plate was then blocked by incubation with 1% BSA in PBS at 37 °C for 1 h. After blocking, the 96-well plate was washed 3 times with PBST (PBS containing 0.05% Tween20). The serially diluted anti-Nectin-4 chimeric antibodies (i.e., antibody 23-H4F3 and antibody 6-B9B6) were prepared in a binding buffer (PBS containing 0.05% Tween20 and 0.5% BSA) and co-incubated with the Nectin family proteins immobilized on the 96-well plate at 37 °C for 1 h. After incubation, the 96-well plate was washed 3 times with PBST, and the cells were co-incubated at 37 °C for 1 h with peroxidase-labeled goat anti-human Fc IgG (Jackson Immuno Research, Cat. No. 109-035-098) diluted at 1/25000 in the binding buffer, washed again, and subjected to a chromogenic reaction with TMB. The reaction was terminated using 1 M H₂SO₄.

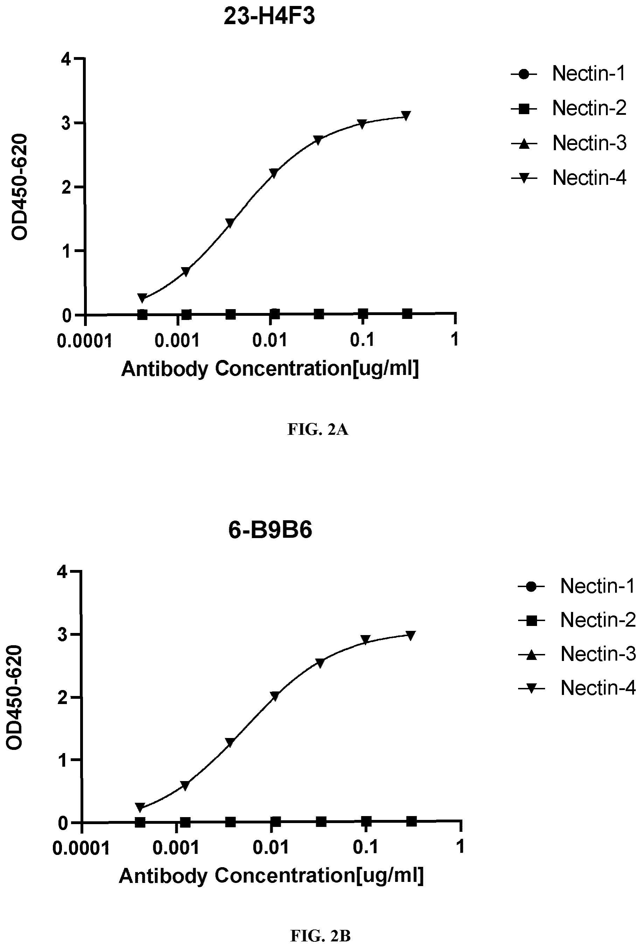

Representative binding curves for the binding of antibody 23-H4F3 and antibody 6-B9B6 to human Nectin family proteins are shown in FIG. 2A and FIG. 2B, respectively.

As can be seen from FIG. 2A and FIG. 2B, antibody 23-H4F3 and antibody 6-B9B6 specifically bound to human Nectin-4 only, and did not bind to Nectin-1, Nectin-2, and Nectin-3.

### Example 3. Humanization of Murine Anti-Nectin-4 Antibodies and Characterization Thereof

### 3.1 Humanization of murine anti-Nectin-4 antibodies

The antibody 23-H4F3 obtained in Example 2.1 was humanized. Specifically, the sequence of antibody 23-H4F3 was searched and aligned in the IMGT database, thereby obtaining a human germline gene sequence IGHV1-3*01 with high homology to the heavy chain variable region of antibody 23-H4F3 for use as a humanized framework of the heavy chain variable region, and obtaining human germline gene sequences IGKV1-6*01 and IGKV4-1*01 with high homology to the light chain variable region for use as humanized frameworks of the light chain variable region. The CDRs in the heavy chain variable region and the light chain variable region of antibody 23-H4F3 were grafted into the corresponding humanized frameworks to form humanized antibody 23-H4F3 variable regions. In order to maintain the affinity of the humanized antibodies for Nectin-4, the humanized antibody variable regions obtained were subjected to back mutation.

The sequences of the humanized heavy chain variable regions and the humanized light chain variable regions were obtained. The variable region amino acid sequences were sent to General Biology System (Anhui) Co., Ltd. for codon optimization and gene synthesis. The genes encoding the humanized VH regions were inserted into an expression vector pcDNA3.1(+) comprising a gene encoding a human IgG1 heavy chain constant region (SEQ ID NO: 7) to obtain plasmids encoding the full-length heavy chains of the anti-Nectin-4 humanized antibodies. The genes encoding the humanized VL regions were inserted into an expression vector pcDNA3.1(+) comprising a gene encoding a human κ light chain constant region (SEQ ID NO: 8) to obtain plasmids encoding the full-length light chains of the anti-Nectin-4 humanized antibodies. The names and the heavy chain variable region sequences of the heavy chains, as well as the names and the light chain variable region sequences of the light chains, of the murine antibody 23-H4F3 after humanization are listed in Table 4.

**Table 4. Names and variable region sequences of heavy chains and light chains of humanized antibodies**

| Heavy chain name | Heavy chain variable region |
|---|---|
| huH4F3H-1 (SEQ ID NO: 25) | SEQ ID NO: 18 |
| huH4F3H-2 (SEQ ID NO: 26) | SEQ ID NO: 19 |

| Light chain name | Light chain variable region |
|---|---|
| huH4F3L-a1 (SEQ ID NO: 27) | SEQ ID NO: 20 |
| huH4F3L-b1 (SEQ ID NO: 28) | SEQ ID NO: 21 |
| huH4F3L-c1 (SEQ ID NO: 29) | SEQ ID NO: 22 |
| huH4F3L-A (SEQ ID NO: 30) | SEQ ID NO: 23 |
| huH4F3L-B (SEQ ID NO: 31) | SEQ ID NO: 24 |

### 3.2 Expression and purification of humanized anti-Nectin-4 antibodies

Similarly as described in Example 2.1, the plasmids encoding the full-length heavy chains of the anti-Nectin-4 humanized antibodies and the plasmids encoding the full-length light chains of the anti-Nectin-4 humanized antibodies were combined (Table 5) and co-transfected into ExpiCHO-S cells to express the respective anti-Nectin-4 humanized antibodies, and the corresponding purification was performed as described in Example 2.1. The purified anti-Nectin-4 humanized antibodies were concentrated, and subjected to sterile filtration. The purities of the anti-Nectin-4 humanized antibodies were detected by SDS-PAGE and size exclusion chromatography (SEC).

**Table 5. Expression combinations of humanized antibodies**

| Name of humanized antibody | Heavy chain | Light chain |
|---|---|---|
| huH4F3-1 | huH4F3H-1 | huH4F3L-a1 |
| huH4F3-2 | huH4F3H-1 | huH4F3L-b1 |
| huH4F3-3 | huH4F3H-1 | huH4F3L-c1 |
| huH4F3-4 | huH4F3H-1 | huH4F3L-A |
| huH4F3-5 | huH4F3H-1 | huH4F3L-B |
| huH4F3-6 | huH4F3H-2 | huH4F3L-a1 |
| huH4F3-7 | huH4F3H-2 | huH4F3L-b1 |
| huH4F3-8 | huH4F3H-2 | huH4F3L-c1 |
| huH4F3-9 | huH4F3H-2 | huH4F3L-A |
| huH4F3-10 | huH4F3H-2 | huH4F3L-B |

### 3.3 Physicochemical analysis of humanized anti-Nectin-4 antibodies

For the humanized anti-Nectin-4 antibodies obtained, the purities of the antibodies in Table 5 were determined by size exclusion chromatography. Specifically, samples of the humanized anti-Nectin-4 antibodies in Table 5 were loaded at 20 µg onto a TSK G3000SWXL column using 100 mM sodium phosphate + 100 mM Na₂SO₄ (pH 7.0) as a running buffer for 30 min. The collected effluent was measured using the Agilent 1220 HPLC system and the data were analyzed using the OpenLAB software. The results indicate that the purities of the humanized anti-Nectin-4 antibodies in Table 5 were greater than 90%, and the antibodies could be used in the next step. 3.4 Binding of humanized anti-Nectin-4 antibodies to tumor cell lines and engineered cells expressing cynomolgus monkey Nectin-4

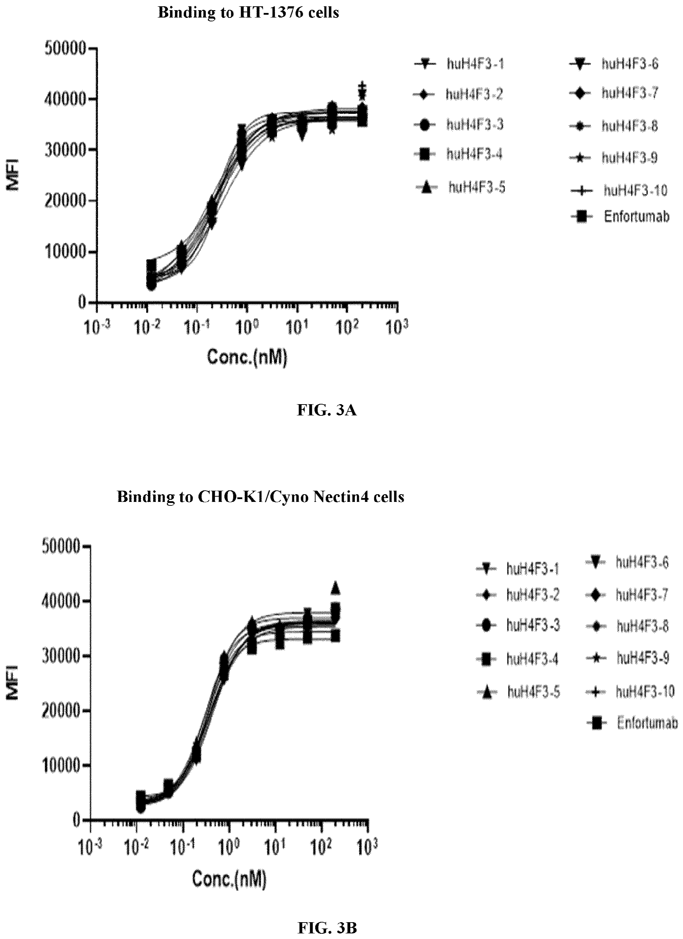

Whether the anti-Nectin-4 humanized antibodies in the present disclosure were able to bind to the human or cynomolgus monkey Nectin-4 protein stably expressed on the cell surface was determined by a cell binding experiment. The specific assay method was as described in Example 2.2, except that the antibodies used were the humanized anti-Nectin-4 antibodies in Table 5. The results are shown in FIG. 3A and FIG. 3B.

FIG. 3A and FIG. 3B show that all the anti-Nectin-4 humanized antibodies in Table 5 were able to bind to HT-1376 cells and cells expressing the cynomolgus monkey Nectin-4 protein, and the binding abilities of the anti-Nectin-4 humanized antibodies were comparable to that of the control antibody enfortumab.

### Example 4. Preparation and Affinity Assay of Anti-Nectin-4×4-1BB Bispecific Antibodies 4.1 Construction of bispecific antibodies

In this example, anti-Nectin-4×4-1BB bispecific antibodies were constructed. The anti-Nectin-4 moiety was derived from chimeric antibodies 23-H4F3 or 32-A12C7 in Example 2.1 or humanized antibody huH4F3-5 in Example 3.2. The anti-4-1BB moiety had a heavy chain variable region set forth in SEQ ID NO: 32 and a light chain variable region set forth in SEQ ID NO: 33. In the present application, the anti-Nectin-4×4-1BB bispecific antibodies were obtained using a standard construction method. The amino acid sequences of the antibodies were listed in the sequence listing, and sequentially comprise, from the N-terminus to the C-terminus, a sequence derived from the anti-Nectin-4 antibody and an anti-4-1BB single-chain antibody moiety linked to the C-terminus of the anti-Nectin-4 antibody Fc region.

Specifically, anti-Nectin-4×4-1BB bispecific antibody molecules were constructed as follows. Heavy chain of anti-Nectin-4×4-1BB bispecific antibody: A nucleotide sequence encoding the heavy chain variable region of the anti-Nectin-4 antibody was amplified and cloned into a pcDNA3.1(+) vector of a human IgG1 mutant heavy chain constant region (SEQ ID NO: 34); the anti-4-1BB VH and VL were amplified using antibody P4B-3 (see CN114555638A) as a template, and an encoding nucleotide sequence of the anti-4-1BB ScFv (i.e., VH-(G₄S)₃ linker-VL) was linked to the C-terminus of the heavy chain constant region of the pcDNA3.1(+) vector comprising the nucleotide sequences encoding the heavy chain variable region of the anti-Nectin-4 antibody and the human IgG1 mutant heavy chain constant region (SEQ ID NO: 34). To prevent cleavage and substitution, the terminal lysine of the Fc was substituted with alanine. The expression vector of the anti-Nectin-4×4-1BB heavy chain was obtained. The anti-Nectin-4×4-1BB heavy chain sequentially comprises, from the N-terminus to the C-terminus, the anti-Nectin-4 heavy chain (with terminal K mutated to A) and the anti-4-1BB ScFv. The heavy chains of the chimeric bispecific antibodies were obtained, i.e., the H4F3-41BB heavy chain (SEQ ID NO: 35), the A12C7-41BB heavy chain (SEQ ID NO: 36), and the humanized bispecific antibody huH4F3-5-41BB heavy chain (SEQ ID NO: 37).

Bispecific antibody light chains: The corresponding light chains of the bispecific antibodies are shown in Table 6.

**Table 6. Expression of chimeric/humanized bispecific antibodies**

| Name of bispecific antibody | Heavy chain | Light chain |
|---|---|---|
| H4F3-41BB | SEQ ID NO: 35 | 23-H4F3-L (SEQ ID NO: 12) |
| A12C7-41BB | SEQ ID NO: 36 | A12C7-L (SEQ ID NO: 16) |
| huH4F3-5-41BB | SEQ ID NO: 37 | huH4F3L-B (SEQ ID NO: 31) |

The obtained heavy chain and light chain expression vectors (plasmids respectively comprising the H4F3-41BB/A12C7-41BB/huH4F3-5-41BB heavy chain-encoding nucleic acids and plasmids respectively comprising 23-H4F3-L/A12C7-L/huH4F3L-B light chain-encoding nucleic acids) were co-transfected into ExpiCHO-S cells for expression under appropriate conditions to obtain bispecific antibody proteins, and the expression, purification, and preliminary analysis steps were the same as those in Example 2.1.

### 4.2 Assay on affinity of bispecific antibodies for human/cynomolgus monkey Nectin-4 and 4-1BB

In this experiment, according to the manufacturer's instructions, ForteBio Octet RED96e was used to assay the binding affinity of the huH4F3-5-41BB bispecific antibody for the human Nectin-4 protein (ACRO Biosystems, Cat. No. NE4-H52H3), the cynomolgus monkey Nectin-4 protein (KACTUS, Cat. No. NEC-CM104), the human 4-1BB protein (Sino Biological, Cat. No. 10041-H08H), or the cynomolgus monkey 4-1BB protein (Sino Biological, Cat. No. 90847-K08H).

Briefly, an AHC sensor (ForteBio, Cat. No. 18-5060) was pre-equilibrated in a running buffer (1× PBS, XiGene, Cat. No. XG3650, containing 0.02% Tween20, pH 7.0) at room temperature for 10 min. In a 96-well plate, the kinetic assay was performed according to the following steps:
a) equilibrating the baseline with the running buffer for 180 s;
b) adding each bispecific antibody diluted with the running buffer at a final concentration of 5 µg/mL and immobilizing for 200 s;
c) equilibrating the baseline with the running buffer for 300 s; and
d1) adding a human Nectin-4 protein or cynomolgus monkey Nectin-4 protein diluted with the running buffer to each well at the following concentrations: 100 nM, 50 nM, 25 nM, 12.5 nM, 6.25 nM, 3.13 nM, and 1.56 nM, binding for 200 s, and dissociating for 600 s; the experimental data were fitted and calculated using a Fortebio Data Analysis software 1:1 binding model; or
d2) adding a human 4-1BB protein or cynomolgus monkey 4-1BB protein diluted with the running buffer to each well at the following concentrations: 400 nM, 200 nM, 100 nM, 50 nM, 25 nM, 12.5 nM, and 6.25 nM, binding for 240 s, and dissociating for 400 s; and binding for 240 s, and dissociating for 600 s; the experimental data were fitted and calculated using a Fortebio Data Analysis software 1:1 binding model.

Table 7 summarizes the binding affinity of the huH4F3-5-41BB bispecific antibody for the human Nectin-4 protein, the cynomolgus monkey Nectin-4 protein, the human 4-1BB protein, or the cynomolgus monkey 4-1BB protein.

**Table 7. Binding affinity of huH4F3-5-41BB bispecific antibody for Nectin-4 or 4-1BB protein**

| Antigen | K_{D} (M) | Kon (1/Ms) | Kdis (1/s) |
|---|---|---|---|
| Human Nectin-4 protein | 2.96E-10 | 3.14E+05 | 9.29E-05 |
| Cynomolgus monkey Nectin-4 protein | 5.88E-09 | 3.07E+05 | 1.80E-03 |
| Human 4-1BB protein | 5.71E-08 | 9.96E+04 | 5.68E-03 |
| Cynomolgus monkey 4-1BB protein | 8.47E-09 | 6.39E+04 | 5.41E-04 |

Table 7 indicates that the bispecific antibody of the present disclosure was able to specifically bind to the human Nectin-4 protein, the cynomolgus monkey Nectin-4 protein, the human 4-1BB protein, or the cynomolgus monkey 4-1BB protein. The anti-Nectin-4×4-1BB bispecific antibody obtained in the present disclosure had a K_{D} value for targeting the human Nectin-4 protein two orders of magnitude higher than a K_{D} value for targeting the human 4-1BB protein. The anti-Nectin-4×4-1BB bispecific antibody targeting human Nectin-4 with high affinity can effectively reduce the side effects of targeting human 4-1BB.

### Example 5. Activation of 4-1BB Signaling Pathway by Humanized Bispecific Antibodies

An HEK293 cell line (ATCC, Cat. No. CRL-1573) was maintained in a DMEM culture medium containing 10% FBS in a humidified incubator with 5% CO₂ at 37 °C. A plasmid of a nucleic acid sequence encoding human 4-1BB (NP_001552.2) and pGL4.32[luc2P/NF-κB-RE/Hygro] (Promega, Cat. No. E849A) were co-transfected into HEK293 cells using a Lipofectamine^{™} 2000 transfection reagent (Invitrogen, Cat. No. 11668019), and after screening with puromycin (Gibco, Cat. No. A1113802) and hygromycin (Gibco, Cat. No. 10687010), clones stably expressing human 4-1BB and NF-κB-Luc were obtained by limited dilution and designated as HEK293/NF-κB-Luc/4-1BB cells.

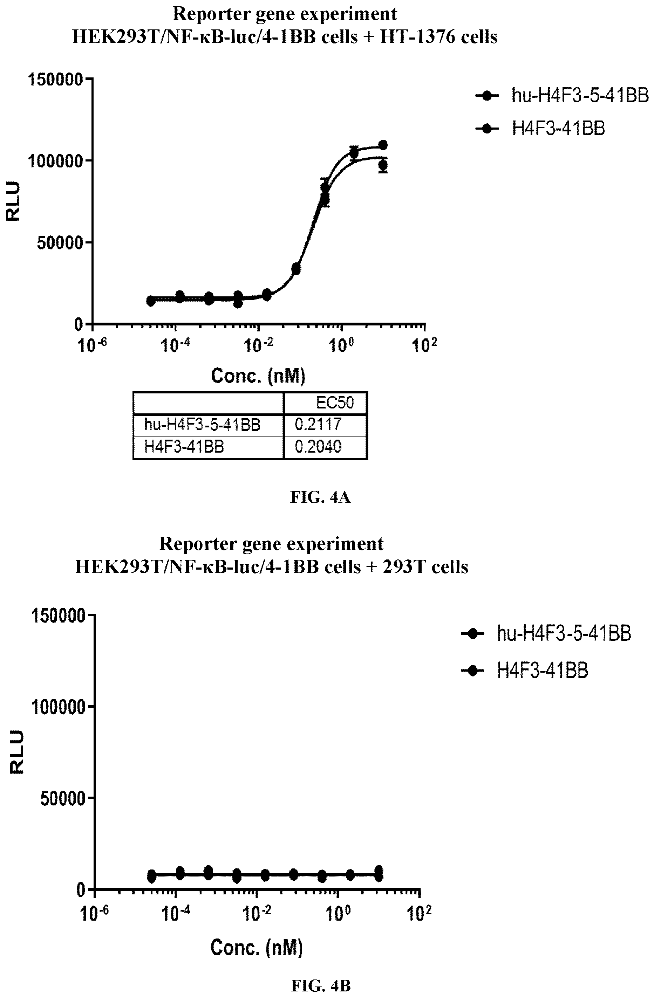

The HEK293/NF-κB-Luc/4-1BB cells were seeded into a 384-well plate (20000 cells/well, 20 µL). HT-1376 cells or 293T cells (not expressing Nectin-4) (ATCC, Cat. No. CRL-1573) were then seeded in the 384-well plate (10000 cells/well, 20 µL). The antibodies were diluted in a 5-fold gradient (at final concentrations starting from 10 nM, 5-fold dilution, 9 points) using a DMEM culture medium containing 10% FBS, and the antibody dilutions were added at 10 µL/well. The culture plate was incubated in an incubator with 5% CO₂ at 37 °C for 6 h. After incubation, 30 µL of a One-Glo^{™} reagent (Promega, Cat. No. E6130) was added to the wells of the assay plate and luminescence was measured using a luminescence plate reader (Tecan F200 Pro). The results are shown in FIG. 4A and FIG. 4B.

As can be seen from FIG. 4A and FIG. 4B, only in the presence of the Nectin-4 protein, the huH4F3-5-41BB bispecific antibody and the H4F3-41BB bispecific antibody were able to activate the human 4-1BB signaling pathway and further activate NF-κB, and the activation showed a dose-dependent trend in the reporter gene system; while in the absence of the Nectin-4 protein, neither the huH4F3-5-41BB bispecific antibody nor the H4F3-41BB bispecific antibody was able to activate the human 4-1BB signaling pathway.

### Example 6. In Vivo Efficacy of Anti-Nectin-4×4-1BB Bispecific Antibodies in MC38-hNectin4 Tumor Model

The *in vivo* anti-tumor effect of the anti-Nectin-4×4-1BB bispecific antibodies was studied in an MC38-hNectin4 tumor model.

Humanized mice C57BL/6-Tnfrsf9tm1 (TNFRSF9)/Bcgen expressing the extracellular portion of human 4-1BB were purchased from Biocytogen Jiangsu Co., Ltd.

Mouse colon cancer MC38 cells were purchased from Shunran (Shanghai) Biotechnology Co., Ltd., and the MC38 cells were genetically engineered to overexpress human Nectin-4 by Biocytogen Pharmaceuticals (Beijing) Co., Ltd. The cells were designated as MC38-hNectin4. The cells were cultured in an incubator with 5% CO₂ at 37 °C, and the culture medium was a DMEM culture medium containing 10% inactivated fetal bovine serum as an ingredient.

MC38-hNectin4 cells were subcutaneously inoculated to the right dorsal side of thirty two C57BL/6-Tnfrsf9tm1 (TNFRSF9)/Bcgen mice at 5 × 10⁵ cells/0.1 mL PBS/mouse. When the average tumor volume reached about 100 mm³, 20 appropriate mice were selected for study and randomly divided into 4 experimental groups (5 mice per group) based on the mouse tumor volume and body weight. On days 0, 3, 7, 10, 14, 17, 21, and 24 after grouping, the blank control (PBS) (group G1), the A12C7-41BB bispecific antibody (5 mg/kg) (group G2), the H4F3-41BB bispecific antibody (5 mg/kg) (group G3), and an equimolar amount of the fusion protein Fc-41BB (SEQ ID NO: 38) (2.6 mg/kg) (group G4) were administered intraperitoneally to the mice, respectively. The fusion protein Fc-41BB was in a double-stranded form and was prepared by the method as described in Example 2.1. During the experiment, the maximum length of the major axis (L) and the maximum length of the minor axis (W) of tumors in tumor-bearing mice were measured twice weekly using a vernier caliper, and the tumor volume was calculated using the following formula: V = L×W²/2.

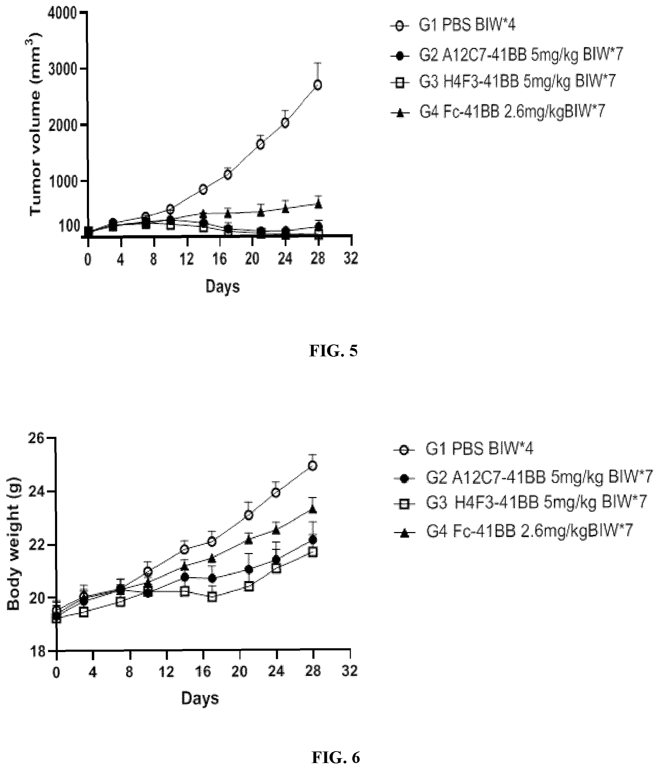

Treatment with anti-Nectin-4×4-1BB bispecific antibodies A12C7-41BB and H4F3-41BB and treatment with the fusion protein Fc-41BB resulted in significant tumor growth inhibition as compared with the PBS group, and the A12C7-41BB group and the H4F3-41BB group exhibited higher tumor growth inhibition rates (TGI rates) as compared with the Fc-41BB group (97.5% and 102.8%, respectively, relative to 81.8% of the Fc-41BB control group). At the end of the experiment, 3 mice survived in the A12C7-41BB bispecific antibody group and 2 mice survived in the H4F3-41BB bispecific antibody group. The results are shown in FIG. 5 and Table 8 below.

**Table 8. In vivo growth inhibition of MC38-hNectin4 tumors**

| Group | Dose | Number of animals (N) | Tumor volume (mm³)^{a} (on day 28 after first administration) | TGI(%)^{b} | P^{c} |
|---|---|---|---|---|---|
| Blank control (PBS) | / | 5 | 2687±389 | - | / |
| A12C7-41BB | 5 mg/kg | 5 | 165±113 | 97.5% | ****<0.0001 |
| H4F3-41BB | 5 mg/kg | 5 | 27±12 | 102.8% | ****<0.0001 |
| Fc-41BB | 2.6 mg/kg | 5 | 570±142 | 81.8% | ****<0.0001 |

| | | | | | |
|---|---|---|---|---|---|
| Note: a. Mean + standard error; b. TGI = [1 - (change in tumor volume of administration group/change in tumor volume of control group)] × 100%; c. Tumor volumes in the administration group and the PBS control group were statistically analyzed (One-way ANOVA) on day 28 of group administration; ****p < 0.0001. | | | | | |

Throughout the course of the experiment, the experimental animals maintained good mobility and feeding status during the administration period, and the body weight of the animals in the G1-G4 groups increased to some extent, indicating that the test substances were well tolerated by the animals. The body weight changes of all animals are shown in FIG. 6 and Table 9.

**Table 9. Effect of test substances on mouse body weight**

| Group | Body weight (g) ^{a} | | | Body weight change (g) on day 28 after administration |
|---|---|---|---|---|
| | Before administration | Day 28 of group administration | p^{b} | |
| Blank control (PBS) | 19.5±0.4 | 24.9±0.4 | - | +5.4 |
| A12C7-41BB | 19.3±0.5 | 22.1±0.7 | 0.0272 | +2.8 |
| H4F3-41BB | 19.2±0.5 | 21.7±0.7 | 0.0042 | +2.4 |
| Fc-41BB | 19.4±0.3 | 23.3±0.4 | 0.4398 | +3.9 |

| | | | | |
|---|---|---|---|---|
| Note: a. Mean ± standard error; b. Body weights in the administration groups and the PBS control group were statistically analyzed (One-way ANOVA) on day 28 of group administration. | | | | |

### Exemplary sequences

| **Sequence No.** | **Description** | **Amino acid sequence** |
|---|---|---|
| SEQ ID NO: 1 | Antibody 23-H4F3 Heavy chain variable region (VH) | QVQLQQSGPELVKPGASVRISCKASGYTFTSYYIHWV KQRPGQGLEWIGWIYPGNVNTKYNEKFKGKATLTA DKSSSTAYMQLISLTSEDSAVYFCARGVYYFDFWGQ GTTLTVSS |
| SEQ ID NO: 2 | Antibody 23-H4F3 Light chain variable region (VL) | SIVMTQTPKFLLVSAGDRVTLTCKASQSVSNDVAWF QQKPGQSPKLLIYYALNRYTGVPDRFTGSGYGTDFTF TISTVQAEDLAVYFCQQDYSSPYTFGGGTKLEIK |
| SEQ ID NO: 3 | Antibody 6-B9B6 Heavy chain variable region (VH) | EVHLVESGGGLVQPGGSLKLSCAASGFTFSSYGMSW VRQTPDKRLELVATINNIGGSTFPPDSVKGRFTVSRD NAKNTLYLQMSSLKSEDTAMYYCARAYDYVPFAYW GQGTLVTVSA |
| SEQ ID NO: 4 | Antibody 6-B9B6 Light chain variable region (VL) | DIQMTQSSSYLSVSLGGRVTITCKASDHINNWLAWY QQKPGNAPRLLISGATSLETGVPSRFSGSGSGKDYAL SIASLQTEDVATYYCQQYWSTPFTFGSGTKLEIK |
| SEQ ID NO: 5 | Antibody 32-A12C7 Heavy chain variable region (VH) | QVQLQQPGAELVMPGASVKMSCKASGYTFTDYWM HWVKQRPGQGLEWIGAIDTSDSYTSYNQKFKGKATL TVDESSSTAYMQLSSLTSEDSAVYYCARWGSTMITW GYGMDNWGQGTSVTVSS |
| SEQ ID NO: 6 | Antibody 32-A12C7 Light chain variable region (VL) | DIQMTQTTSSLSASLGDRVTISCRASQDINNYLNWYQ QKPDGTVKLLIYYTSRLHSGVPSRFSGSGSGTDYSLTI SNLEQEDIATYFCQQGNTLPWTFGGGTKLEIK |
| SEQ ID NO: 7 | Human IgG1 heavy chain constant region | |
| SEQ ID NO: 8 | κ light chain constant region | |
| SEQ ID NO: 9 | Enfortumab | EVQLVESGGGLVQPGGSLRLSCAASGFTFSSYNMNW |
| | Heavy chain | VRQAPGKGLEWVSYISSSSSTIYYADSVKGRFTISRDN AKNSLSLQMNSLRDEDTAVYYCARAYYYGMDVWG QGTTVTVSSASTKGPSVFPLAPSSKSTSGGTAALGCL VKDYFPEPVTVSWNSGALTSGVHTFPAVLQSSGLYSL SSVVTVPSSSLGTQTYICNVNHKPSNTKVDKKVEPKS CDKTHTCPPCPAPELLGGPSVFLFPPKPKDTLMISRTP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSD GSFFLYSKLTVDKSRWQQGNVFSCSVMHEALHNHY TQKSLSLSPGK |
| SEQ ID NO: 10 | Enfortumab Light chain | DIQMTQSPSSVSASVGDRVTITCRASQGISGWLAWYQ QKPGKAPKFLIYAASTLQSGVPSRFSGSGSGTDFTLTI SSLQPEDFATYYCQQANSFPPTFGGGTKVEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| SEQ ID NO: 11 | Antibody 23-H4F3 Heavy chain | QVQLQQSGPELVKPGASVRISCKASGYTFTSYYIHWV KQRPGQGLEWIGWIYPGNVNTKYNEKFKGKATLTA DKSSSTAYMQLISLTSEDSAVYFCARGVYYFDFWGQ GTTLTVSSASTKGPSVFPLAPSSKSTSGGTAALGCLV KDYFPEPVTVSWNSGALTSGVHTFPAVLQSSGLYSLS SVVTVPSSSLGTQTYICNVNHKPSNTKVDKKVEPKSC DKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQ KSLSLSPGK |
| SEQ ID NO: 12 | Antibody 23-H4F3 Light chain | SIVMTQTPKFLLVSAGDRVTLTCKASQSVSNDVAWF QQKPGQSPKLLIYYALNRYTGVPDRFTGSGYGTDFTF TISTVQAEDLAVYFCQQDYSSPYTFGGGTKLEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |
| SEQ ID NO: 13 | Antibody 6-B9B6 Heavy chain | EVHLVESGGGLVQPGGSLKLSCAASGFTFSSYGMSW VRQTPDKRLELVATINNIGGSTFPPDSVKGRFTVSRD NAKNTLYLQMSSLKSEDTAMYYCARAYDYVPFAYW GQGTLVTVSAASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPELLGGPSVFLFPPKPKDTLMISRT PEVTCVVVDVSHEDPEVKFNWYVDGVEVHNAKTKP REEQYNSTYRVVSVLTVLHQDWLNGKEYKCKVSNK ALPAPIEKTISKAKGQPREPQVYTLPPSRDELTKNQVS LTCLVKGFYPSDIAVEWESNGQPENNYKTTPPVLDSD GSFFLYSKLTVDKSRWQQGNVFSCSVMHEALHNHY TQKSLSLSPGK |
| SEQ ID NO: 14 | Antibody 6-B9B6 Light chain | DIQMTQSSSYLSVSLGGRVTITCKASDHINNWLAWY QQKPGNAPRLLISGATSLETGVPSRFSGSGSGKDYAL SIASLQTEDVATYYCQQYWSTPFTFGSGTKLEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |
| SEQ ID NO: 15 | Antibody 32-A12C7 Heavy chain | QVQLQQPGAELVMPGASVKMSCKASGYTFTDYWM HWVKQRPGQGLEWIGAIDTSDSYTSYNQKFKGKATL TVDESSSTAYMQLSSLTSEDSAVYYCARWGSTMITW GYGMDNWGQGTSVTVSSASTKGPSVFPLAPSSKSTS GGTAALGCLVKDYFPEPVTVSWNSGALTSGVHTFPA VLQSSGLYSLSSVVTVPSSSLGTQTYICNVNHKPSNT KVDKKVEPKSCDKTHTCPPCPAPELLGGPSVFLFPPK PKDTLMISRTPEVTCVVVDVSHEDPEVKFNWYVDGV EVHNAKTKPREEQYNSTYRVVSVLTVLHQDWLNGK EYKCKVSNKALPAPIEKTISKAKGQPREPQVYTLPPSR DELTKNQVSLTCLVKGFYPSDIAVEWESNGQPENNY KTTPPVLDSDGSFFLYSKLTVDKSRWQQGNVFSCSV MHEALHNHYTQKSLSLSPGK* |
| SEQ ID NO: 16 | Antibody 32-A12C7 Light chain | DIQMTQTTSSLSASLGDRVTISCRASQDINNYLNWYQ QKPDGTVKLLIYYTSRLHSGVPSRFSGSGSGTDYSLTI SNLEQEDIATYFCQQGNTLPWTFGGGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| SEQ ID NO: 17 | Extracellular and transmembrane amino acid sequences of cynomolgus monkey Nectin-4 | MPLSLGAEMWGPEAWLLLLLLLASFTGRCPAGELET SDVVTVVLGQDAKLPCFYRGDSGEQVGQVAWARAD AGEGAQELALLHSKYGLHVSPAYEGRVEQPPPPRNP LDGSVLLRNAVQADEGEYECRVSTFPAGSFQARLRL RVLVPPLPSLNPGPALEEGQGLTLAASCTAEGSPAPS VTWDTEVKGTTSSRSFKHSRSAAVTSEFHLVPSRSMN GQPLTCVVSHPGLLQDQRITHILHVSFLAEASVRGLE DQNLWHVGREGAMLKCLSEGQPPPSYNWTRLDGPL PSGVRVDGDTLGFPPLTTEHSGIYVCHVSNEFSSRDS QVTVDVLDPQEDSGKQVDLVSASVVVVGVIAALLFC LLVVVVVL |
| SEQ ID NO: 18 | huH4F3H-1 Heavy chain variable region | QVQLVQSGAEVKKPGASVKVSCKASGYTFTSYYIHW VRQAPGQRLEWMGWIYPGNVNTKYSQKFQGRVTLT RDKSASTAYMELSSLRSEDTAVYYCARGVYYFDFW GQGTLVTVSS |
| SEQ ID NO: 19 | huH4F3H-2 Heavy chain variable region | QVQLVQSGAEVKKPGASVKVSCKASGYTFTSYYIHW VRQAPGQRLEWMGWIYPGNVNTKYSQKFQGRVTLT RDKSASTAYMELSSLRSEDTAVYFCARGVYYFDFWG QGTLVTVSS |
| SEQ ID NO: 20 | huH4F3L-a1 Light chain variable region | AIQMTQSPSSLSASVGDRVTITCRASQSVSNDVGWYQ QKPGKAPKLLIYYALNLQSGVPSRFSGSGSGTDFTLTI SSLQPEDFATYYCQQDYSSPYTFGQGTKLEIK |
| SEQ ID NO: 21 | huH4F3L-b1 Light chain variable region | AIQMTQSPSSLSASVGDRVTITCRASQSVSNDVGWYQ QKPGKAPKLLIYYALNRYTGVPSRFSGSGSGTDFTLTI SSLQPEDFATYYCQQDYSSPYTFGQGTKLEIK |
| SEQ ID NO: 22 | huH4F3L-c1 Light chain variable region | AIQMTQSPSSLSASVGDRVTITCRASQSVSNDVGWFQ QKPGKAPKLLIYYALNRYTGVPSRFSGSGSGTDFTFTI SSLQPEDFATYYCQQDYSSPYTFGQGTKLEIK |
| SEQ ID NO: 23 | huH4F3L-A Light chain variable region | DIVMTQSPDSLAVSLGERATINCKASQSVSNDVAWY QQKPGQPPKLLIYYALNRESGVPDRFSGSGSGTDFTL TISSLQAEDVAVYYCQQDYSSPYTFGQGTKLEIK |
| SEQ ID NO: 24 | huH4F3L-B Light chain variable region | DIVMTQSPDSLAVSLGERATINCKSSQSVSNDVAWY QQKPGQPPKLLIYYALNRESGVPDRFSGSGSGTDFTL TISSLQAEDVAVYYCQQDYSSPYTFGQGTKLEIK |
| SEQ ID NO: 25 | huH4F3H-1 Heavy chain | QVQLVQSGAEVKKPGASVKVSCKASGYTFTSYYIHW VRQAPGQRLEWMGWIYPGNVNTKYSQKFQGRVTLT RDKSASTAYMELSSLRSEDTAVYYCARGVYYFDFW GQGTLVTVSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPELLGGPSVFLFPPKPKDTLMISRT PEVTCVVVDVSHEDPEVKFNWYVDGVEVHNAKTKP REEQYNSTYRVVSVLTVLHQDWLNGKEYKCKVSNK ALPAPIEKTISKAKGQPREPQVYTLPPSRDELTKNQVS LTCLVKGFYPSDIAVEWESNGQPENNYKTTPPVLDSD GSFFLYSKLTVDKSRWQQGNVFSCSVMHEALHNHY TQKSLSLSPGK |
| SEQ ID NO: 26 | huH4F3H-2 Heavy chain | QVQLVQSGAEVKKPGASVKVSCKASGYTFTSYYIHW VRQAPGQRLEWMGWIYPGNVNTKYSQKFQGRVTLT RDKSASTAYMELSSLRSEDTAVYFCARGVYYFDFWG QGTLVTVSSASTKGPSVFPLAPSSKSTSGGTAALGCL VKDYFPEPVTVSWNSGALTSGVHTFPAVLQSSGLYSL SSVVTVPSSSLGTQTYICNVNHKPSNTKVDKKVEPKS CDKTHTCPPCPAPELLGGPSVFLFPPKPKDTLMISRTP EVTCVVVDVSHEDPEVKFNWYVDGVEVHNAKTKPR EEQYNSTYRVVSVLTVLHQDWLNGKEYKCKVSNKA LPAPIEKTISKAKGQPREPQVYTLPPSRDELTKNQVSL TCLVKGFYPSDIAVEWESNGQPENNYKTTPPVLDSD GSFFLYSKLTVDKSRWQQGNVFSCSVMHEALHNHY TQKSLSLSPGK |
| SEQ ID NO: 27 | huH4F3L-a1 Light chain | AIQMTQSPSSLSASVGDRVTITCRASQSVSNDVGWYQ QKPGKAPKLLIYYALNLQSGVPSRFSGSGSGTDFTLTI SSLQPEDFATYYCQQDYSSPYTFGQGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| SEQ ID NO: 28 | huH4F3L-b1 Light chain | AIQMTQSPSSLSASVGDRVTITCRASQSVSNDVGWYQ QKPGKAPKLLIYYALNRYTGVPSRFSGSGSGTDFTLTI SSLQPEDFATYYCQQDYSSPYTFGQGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| SEQ ID NO: 29 | huH4F3L-c1 Light chain | AIQMTQSPSSLSASVGDRVTITCRASQSVSNDVGWFQ QKPGKAPKLLIYYALNRYTGVPSRFSGSGSGTDFTFTI SSLQPEDFATYYCQQDYSSPYTFGQGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| SEQ ID NO: 30 | huH4F3L-A Light chain | DIVMTQSPDSLAVSLGERATINCKASQSVSNDVAWY QQKPGQPPKLLIYYALNRESGVPDRFSGSGSGTDFTL TISSLQAEDVAVYYCQQDYSSPYTFGQGTKLEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |
| SEQ ID NO: 31 | huH4F3L-B Light chain | DIVMTQSPDSLAVSLGERATINCKSSQSVSNDVAWY QQKPGQPPKLLIYYALNRESGVPDRFSGSGSGTDFTL TISSLQAEDVAVYYCQQDYSSPYTFGQGTKLEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |
| SEQ ID NO: 32 | Anti-4-1BB Heavy chain variable region | QVQLQESGGGLVQPGGSLRLSCAVSGFTFSSYAMHW VRQAPGKCLEWVAVISYDGSKKWYADSVKGRFTISR DNSKNTLYLQMNSLRAEDTAVYYCARNQGSGSYLY YYYMDVWGKGTTVTVSS |
| SEQ ID NO: 33 | Anti-4-1BB Light chain variable region | QSALTQPRSVSGSPGQSVTISCTGTSSDVGGYNYVSW YQQLPGKAPKVIIYEVSNRPSGVSNRFSGSKSGNTAS LTISGVQSEDEADYYCSSYTSSSTFYVFGCGTQLTVL |
| SEQ ID NO: 34 | Human IgG1 mutant heavy chain constant region | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVA VSHEDPEVKFNWYVDGVEVHNAKTKPREEQYNSTY RVVSVLTVLHQDWLNGKEYKCKVSNKALAAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP GK |
| SEQ ID NO: 35 | Bispecific antibody H4F3-41BB Heavy chain | QVQLQQSGPELVKPGASVRISCKASGYTFTSYYIHWV KQRPGQGLEWIGWIYPGNVNTKYNEKFKGKATLTA DKSSSTAYMQLISLTSEDSAVYFCARGVYYFDFWGQ GTTLTVSSASTKGPSVFPLAPSSKSTSGGTAALGCLV KDYFPEPVTVSWNSGALTSGVHTFPAVLQSSGLYSLS SVVTVPSSSLGTQTYICNVNHKPSNTKVDKKVEPKSC DKTHTCPPCPAPEAAGGPSVFLFPPKPKDTLMISRTPE VTCVVVAVSHEDPEVKFNWYVDGVEVHNAKTKPRE EQYNSTYRVVSVLTVLHQDWLNGKEYKCKVSNKAL AAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQ KSLSLSPGAGGGGSGGGGSGGGGSQVQLQESGGGLV QPGGSLRLSCAVSGFTFSSYAMHWVRQAPGKCLEW VAVISYDGSKKWYADSVKGRFTISRDNSKNTLYLQM NSLRAEDTAVYYCARNQGSGSYLYYYYMDVWGKG TTVTVSSGGGGSGGGGSGGGGSQSALTQPRSVSGSP GQSVTISCTGTSSDVGGYNYVSWYQQLPGKAPKVIIY EVSNRPSGVSNRFSGSKSGNTASLTISGVQSEDEADY YCSSYTSSSTFYVFGCGTQLTVL |
| SEQ ID NO: 36 | Bispecific antibody A12C7-41BB Heavy chain | QVQLQQPGAELVMPGASVKMSCKASGYTFTDYWM HWVKQRPGQGLEWIGAIDTSDSYTSYNQKFKGKATL TVDESSSTAYMQLSSLTSEDSAVYYCARWGSTMITW GYGMDNWGQGTSVTVSS ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGGPSVFLFPPKPKDTLMISRTPEVTCVVVA VSHEDPEVKFNWYVDGVEVHNAKTKPREEQYNSTY RVVSVLTVLHQDWLNGKEYKCKVSNKALAAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP GAGGGGSGGGGSGGGGSQVQLQESGGGLVQPGGSL RLSCAVSGFTFSSYAMHWVRQAPGKCLEWVAVISYD GSKKWYADSVKGRFTISRDNSKNTLYLQMNSLRAED TAVYYCARNQGSGSYLYYYYMDVWGKGTTVTVSS GGGGSGGGGSGGGGSQSALTQPRSVSGSPGQSVTISC TGTSSDVGGYNYVSWYQQLPGKAPKVIIYEVSNRPS GVSNRFSGSKSGNTASLTISGVQSEDEADYYCSSYTS SSTFYVFGCGTQLTVL |
| SEQ ID NO: 37 | Bispecific antibody huH4F3-5-41BB Heavy chain | QVQLVQSGAEVKKPGASVKVSCKASGYTFTSYYIHW VRQAPGQRLEWMGWIYPGNVNTKYSQKFQGRVTLT RDKSASTAYMELSSLRSEDTAVYYCARGVYYFDFW GQGTLVTVSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPEAAGGPSVFLFPPKPKDTLMISRT PEVTCVVVAVSHEDPEVKFNWYVDGVEVHNAKTKP REEQYNSTYRVVSVLTVLHQDWLNGKEYKCKVSNK ALAAPIEKTISKAKGQPREPQVYTLPPSRDELTKNQVS LTCLVKGFYPSDIAVEWESNGQPENNYKTTPPVLDSD GSFFLYSKLTVDKSRWQQGNVFSCSVMHEALHNHY TQKSLSLSPGAGGGGSGGGGSGGGGSQVQLQESGGG LVQPGGSLRLSCAVSGFTFSSYAMHWVRQAPGKCLE WVAVISYDGSKKWYADSVKGRFTISRDNSKNTLYLQ MNSLRAEDTAVYYCARNQGSGSYLYYYYMDVWGK GTTVTVSSGGGGSGGGGSGGGGSQSALTQPRSVSGS PGQSVTISCTGTSSDVGGYNYVSWYQQLPGKAPKVII YEVSNRPSGVSNRFSGSKSGNTASLTISGVQSEDEAD YYCSSYTSSSTFYVFGCGTQLTVL |
| SEQ ID NO: 38 | Fc-41BB | DKTHTCPPCPAPEAAGGPSVFLFPPKPKDTLMISRTPE VTCVVVAVSHEDPEVKFNWYVDGVEVHNAKTKPRE EQYNSTYRVVSVLTVLHQDWLNGKEYKCKVSNKAL AAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQ KSLSLSPGKGGGGSGGGGSGGGGSQVQLQESGGGLV QPGGSLRLSCAVSGFTFSSYAMHWVRQAPGKCLEW VAVISYDGSKKWYADSVKGRFTISRDNSKNTLYLQM NSLRAEDTAVYYCARNQGSGSYLYYYYMDVWGKG TTVTVSSGSSSSGSSSSGSSSSQSALTQPRSVSGSPGQS VTISCTGTSSDVGGYNYVSWYQQLPGKAPKVIIYEVS NRPSGVSNRFSGSKSGNTASLTISGVQSEDEADYYCS SYTSSSTFYVFGCGTQLTVL |
| SEQ ID NO: 39 | Extracellular amino acid sequence of human Nectin-4 | MPLSLGAEMWGPEAWLLLLLLLASFTGRCPAGELET SDVVTVVLGQDAKLPCFYRGDSGEQVGQVAWARVD AGEGAQELALLHSKYGLHVSPAYEGRVEQPPPPRNP LDGSVLLRNAVQADEGEYECRVSTFPAGSFQARLRL RVLVPPLPSLNPGPALEEGQGLTLAASCTAEGSPAPS VTWDTEVKGTTSSRSFKHSRSAAVTSEFHLVPSRSMN GQPLTCVVSHPGLLQDQRITHILHVSFLAEASVRGLE DQNLWHIGREGAMLKCLSEGQPPPSYNWTRLDGPLP SGVRVDGDTLGFPPLTTEHSGIYVCHVSNEFSSRDSQ VTVDVLDPQEDSGKQVDLVSAS |
| SEQ ID NO: 40 | Antibody 23-H4F3 | SYYIH |
| | HCDR1; | |
| | huH4F3H-1 HCDR1; | |
| | huH4F3H-2 HCDR1 (Kabat numbering) | |
| SEQ ID NO: 41 | Antibody 23-H4F3 HCDR2 (Kabat numbering) | WIYPGNVNTKYNEKFKG |
| SEQ ID NO: 42 SEQ ID NO: 43 | Antibody 23-H4F3 HCDR3 | GVYYFDF |
| | huH4F3H-1 HCDR3; | |
| | huH4F3H-2 HCDR3 (Kabat numbering) | |
| | Antibody 23-H4F3 LCDR1; | KASQSVSNDVA |
| | huH4F3L-A LCDR1 (Kabat numbering) | |
| SEQ ID NO: 44 | Antibody 23-H4F3 | YALNRYT |
| | LCDR2; | |
| | huH4F3L-b1 LCDR2; | |
| | huH4F3L-c1 LCDR2 (Kabat numbering) | |
| SEQ ID NO: 45 | Antibody 23-H4F3 LCDR3; | QQDYSSPYT |
| | huH4F3L-a1 LCDR3; | |
| | huH4F3L-b1 LCDR3; | |
| | huH4F3L-c1 LCDR3; | |
| | huH4F3L-A LCDR3; | |
| | huH4F3L-B LCDR3 (Kabat numbering) | |
| SEQ ID NO: 46 | Antibody 6-B9B6 HCDR1 (Kabat numbering) | SYGMS |
| SEQ ID NO: 47 | Antibody 6-B9B6 HCDR2 (Kabat numbering) | TINNIGGSTFPPDSVKG |
| SEQ ID NO: 48 | Antibody 6-B9B6 HCDR3 (Kabat numbering) | AYDYVPFAY |
| SEQ ID NO: 49 | Antibody 6-B9B6 LCDR1 (Kabat numbering) | KASDHINNWLA |
| SEQ ID NO: 50 | Antibody 6-B9B6 LCDR2 (Kabat numbering) | GATSLET |
| SEQ ID NO: 51 | Antibody 6-B9B6 LCDR3 (Kabat numbering) | QQYWSTPF |
| SEQ ID NO: 52 | Antibody 32-A12C7 HCDR1 (Kabat numbering) | DYWMH |
| SEQ ID NO: 53 | Antibody 32-A12C7 HCDR2 | AIDTSDSYTSYNQKFKG |
| SEQ ID NO: 54 | Antibody 32-A12C7 HCDR3 (Kabat numbering) | WGSTMITWGYGMDN |
| SEQ ID NO: 55 | Antibody 32-A12C7 LCDR1 (Kabat numbering) | RASQDINNYLN |
| SEQ ID NO: 56 | Antibody 32-A12C7 LCDR2 (Kabat numbering) | YTSRLHS |
| SEQ ID NO: 57 | Antibody 32-A12C7 LCDR3 (Kabat numbering) | QQGNTLPWT |
| SEQ ID NO: 58 | huH4F3H-1 HCDR2; | WIYPGNVNTKYSQKFQG |
| | huH4F3H-2 HCDR2 (Kabat numbering) | |
| SEQ ID NO: 59 | huH4F3L-a1 LCDR1; | RASQSVSNDVG |
| | huH4F3L-b1 LCDR1; | |
| | huH4F3L-c1 LCDR1 (Kabat numbering) | |
| SEQ ID NO: 60 | huH4F3L-B LCDR1 | KSSQSVSNDVA |
| SEQ ID NO: 61 | huH4F3L-a1 LCDR2 (Kabat numbering) | YALNLQS |
| SEQ ID NO: 62 | huH4F3L-A LCDR2; | YALNRES |
| | huH4F3L-B LCDR2 (Kabat numbering) | |
| SEQ ID NO: 63 | Anti-4-1BB HCDR1 (Kabat numbering) | SYAMH |
| SEQ ID NO: 64 | Anti-4-1BB HCDR2 (Kabat numbering) | VISYDGSKKWYADSVKG |
| SEQ ID NO: 65 | Anti-4-1BB HCDR3 (Kabat numbering) | NQGSGSYLYYYYMDV |
| SEQ ID NO: 66 | Anti-4-1BB LCDR1 (Kabat numbering) | TGTSSDVGGYNYVS |
| SEQ ID NO: 67 | Anti-4-1BB LCDR2 (Kabat numbering) | EVSNRPS |
| SEQ ID NO: 68 | Anti-4-1BB LCDR3 (Kabat numbering) | SSYTSSSTFYV |
| SEQ ID NO: 69 | Linker peptide 1 | GGGGSGGGGSGGGGS |
| SEQ ID NO: 70 | Linker peptide 2 | GSSSSGSSSSGSSSS |

The exemplary embodiments of the present disclosure have been described above, and it should be understood by those skilled in the art that these disclosed contents are merely exemplary and that various other substitutions, adaptations, and modifications may be made within the scope of the present disclosure. Therefore, the present disclosure is not limited to the specific embodiments listed herein.

## Claims

1. An isolated antibody specifically binding to a Nectin-4 protein or an antigen-binding fragment of the isolated antibody, comprising:
(a) 3 CDRs in the amino acid sequence of a heavy chain variable region set forth in SEQ ID NO: 1 and 3 CDRs in the amino acid sequence of a light chain variable region set forth in SEQ ID NO: 2; or a variant with one or more CDRs each having no more than 3 amino acid modifications relative to the 6 CDR regions described above;
for example, 3 CDRs in the amino acid sequence of a heavy chain variable region set forth in SEQ ID NO: 18 and 3 CDRs in the amino acid sequence of a light chain variable region set forth in any one of SEQ ID NOs: 20-24; or
3 CDRs in the amino acid sequence of a heavy chain variable region set forth in SEQ ID NO: 19 and 3 CDRs in the amino acid sequence of a light chain variable region set forth in any one of SEQ ID NOs: 20-24;
(b) 3 CDRs in the amino acid sequence of a heavy chain variable region set forth in SEQ ID NO: 3 and 3 CDRs in the amino acid sequence of a light chain variable region set forth in SEQ ID NO: 4; or a variant with one or more CDRs each having no more than 3 amino acid modifications relative to the 6 CDR regions described above; or
(c) 3 CDRs in the amino acid sequence of a heavy chain variable region set forth in SEQ ID NO: 5 and 3 CDRs in the amino acid sequence of a light chain variable region set forth in SEQ ID NO: 6; or a variant with one or more CDRs each having no more than 3 amino acid modifications relative to the 6 CDR regions described above.

2. An isolated antibody specifically binding to a Nectin-4 protein or an antigen-binding fragment of the isolated antibody, comprising, according to Kabat numbering:
(a) an HCDR1 set forth in SEQ ID NO: 40 or a variant of the HCDR1 set forth in SEQ ID NO: 40 having no more than 2 amino acid modifications, an HCDR2 set forth in SEQ ID NO: 41 or a variant of the HCDR2 set forth in SEQ ID NO: 41 having no more than 3 amino acid modifications, and an HCDR3 set forth in SEQ ID NO: 42 or a variant of the HCDR3 set forth in SEQ ID NO: 42 having no more than 2 amino acid modifications; an LCDR1 set forth in SEQ ID NO: 43 or a variant of the LCDR1 set forth in SEQ ID NO: 43 having no more than 3 amino acid modifications, an LCDR2 set forth in SEQ ID NO: 44 or a variant of the LCDR2 set forth in SEQ ID NO: 44 having no more than 3 amino acid modifications, and an LCDR3 set forth in SEQ ID NO: 45 or a variant of the LCDR3 set forth in SEQ ID NO: 45 having no more than 2 amino acid modifications;
(b) an HCDR1 set forth in SEQ ID NO: 46 or a variant of the HCDR1 set forth in SEQ ID NO: 46 having no more than 2 amino acid modifications, an HCDR2 set forth in SEQ ID NO: 47 or a variant of the HCDR2 set forth in SEQ ID NO: 47 having no more than 3 amino acid modifications, and an HCDR3 set forth in SEQ ID NO: 48 or a variant of the HCDR3 set forth in SEQ ID NO: 48 having no more than 2 amino acid modifications; an LCDR1 set forth in SEQ ID NO: 49 or a variant of the LCDR1 set forth in SEQ ID NO: 49 having no more than 3 amino acid modifications, an LCDR2 set forth in SEQ ID NO: 50 or a variant of the LCDR2 set forth in SEQ ID NO: 50 having no more than 3 amino acid modifications, and an LCDR3 set forth in SEQ ID NO: 51 or a variant of the LCDR3 set forth in SEQ ID NO: 51 having no more than 2 amino acid modifications; or
(c) an HCDR1 set forth in SEQ ID NO: 52 or a variant of the HCDR1 set forth in SEQ ID NO: 52 having no more than 2 amino acid modifications, an HCDR2 set forth in SEQ ID NO: 53 or a variant of the HCDR2 set forth in SEQ ID NO: 53 having no more than 3 amino acid modifications, and an HCDR3 set forth in SEQ ID NO: 54 or a variant of the HCDR3 set forth in SEQ ID NO: 54 having no more than 2 amino acid modifications; an LCDR1 set forth in SEQ ID NO: 55 or a variant of the LCDR1 set forth in SEQ ID NO: 55 having no more than 3 amino acid modifications, an LCDR2 set forth in SEQ ID NO: 56 or a variant of the LCDR2 set forth in SEQ ID NO: 56 having no more than 3 amino acid modifications, and an LCDR3 set forth in SEQ ID NO: 57 or a variant of the LCDR3 set forth in SEQ ID NO: 57 having no more than 2 amino acid modifications;
wherein preferably, the isolated antibody or the antigen-binding fragment specifically binding to the Nectin-4 protein comprises, according to Kabat numbering:
(a) an HCDR1 set forth in SEQ ID NO: 40, an HCDR2 set forth in SEQ ID NO: 41, and an HCDR3 set forth in SEQ ID NO: 42; an LCDR1 set forth in SEQ ID NO: 43, an LCDR2 set forth in SEQ ID NO: 44, and an LCDR3 set forth in SEQ ID NO: 45;
an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 59, an LCDR2 set forth in SEQ ID NO: 61, and an LCDR3 set forth in SEQ ID NO: 45;
an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 59, an LCDR2 set forth in SEQ ID NO: 44, and an LCDR3 set forth in SEQ ID NO: 45;
an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 43, an LCDR2 set forth in SEQ ID NO: 62, and an LCDR3 set forth in SEQ ID NO: 45;
an HCDR1 set forth in SEQ ID NO: 40, an HCDR2 set forth in SEQ ID NO: 58, and an HCDR3 set forth in SEQ ID NO: 42; an LCDR1 set forth in SEQ ID NO: 60, an LCDR2 set forth in SEQ ID NO: 62, and an LCDR3 set forth in SEQ ID NO: 45;
(b) an HCDR1 set forth in SEQ ID NO: 46, an HCDR2 set forth in SEQ ID NO: 47, and an HCDR3 set forth in SEQ ID NO: 48; an LCDR1 set forth in SEQ ID NO: 49, an LCDR2 set forth in SEQ ID NO: 50, and an LCDR3 set forth in SEQ ID NO: 51; or
(c) an HCDR1 set forth in SEQ ID NO: 52, an HCDR2 set forth in SEQ ID NO: 53, and an HCDR3 set forth in SEQ ID NO: 54; an LCDR1 set forth in SEQ ID NO: 55, an LCDR2 set forth in SEQ ID NO: 56, and an LCDR3 set forth in SEQ ID NO: 57.

3. The isolated antibody or the antigen-binding fragment of the isolated antibody according to claim 1 or 2, comprising:
(a) a heavy chain variable region and a light chain variable region, wherein the heavy chain variable region comprises the sequence of SEQ ID NO: 1 or a sequence having at least 90%, 91%, 92%, 93%, 94%, 95%, 96%, 97%, 98%, or 99% identity thereto, and the light chain variable region comprises the sequence of SEQ ID NO: 2 or a sequence having at least 90%, 91%, 92%, 93%, 94%, 95%, 96%, 97%, 98%, or 99% identity thereto;
for example, a heavy chain variable region set forth in SEQ ID NO: 1 and a light chain variable region set forth in SEQ ID NO: 2;
a heavy chain variable region set forth in SEQ ID NO: 18 and a light chain variable region set forth in SEQ ID NO: 20;
a heavy chain variable region set forth in SEQ ID NO: 18 and a light chain variable region set forth in SEQ ID NO: 21;
a heavy chain variable region set forth in SEQ ID NO: 18 and a light chain variable region set forth in SEQ ID NO: 22;
a heavy chain variable region set forth in SEQ ID NO: 18 and a light chain variable region set forth in SEQ ID NO: 23;
a heavy chain variable region set forth in SEQ ID NO: 18 and a light chain variable region set forth in SEQ ID NO: 24;
a heavy chain variable region set forth in SEQ ID NO: 19 and a light chain variable region set forth in SEQ ID NO: 20;
a heavy chain variable region set forth in SEQ ID NO: 19 and a light chain variable region set forth in SEQ ID NO: 21;
a heavy chain variable region set forth in SEQ ID NO: 19 and a light chain variable region set forth in SEQ ID NO: 22;
a heavy chain variable region set forth in SEQ ID NO: 19 and a light chain variable region set forth in SEQ ID NO: 23;
a heavy chain variable region set forth in SEQ ID NO: 19 and a light chain variable region set forth in SEQ ID NO: 24;
(b) a heavy chain variable region and a light chain variable region, wherein the heavy chain variable region comprises the sequence of SEQ ID NO: 3 or a sequence having at least 90%, 91%, 92%, 93%, 94%, 95%, 96%, 97%, 98%, or 99% identity thereto, and the light chain variable region comprises the sequence of SEQ ID NO: 4 or a sequence having at least 90%, 91%, 92%, 93%, 94%, 95%, 96%, 97%, 98%, or 99% identity thereto;
for example, a heavy chain variable region set forth in SEQ ID NO: 3 and a light chain variable region set forth in SEQ ID NO: 4; or
(c) a heavy chain variable region and a light chain variable region, wherein the heavy chain variable region comprises the sequence of SEQ ID NO: 5 or a sequence having at least 90%, 91%, 92%, 93%, 94%, 95%, 96%, 97%, 98%, or 99% identity thereto, and the light chain variable region comprises the sequence of SEQ ID NO: 6 or a sequence having at least 90%, 91%, 92%, 93%, 94%, 95%, 96%, 97%, 98%, or 99% identity thereto;
for example, a heavy chain variable region set forth in SEQ ID NO: 5 and a light chain variable region set forth in SEQ ID NO: 6;
wherein preferably, the isolated antibody comprises:
(a) SEQ ID NO: 11 or a heavy chain sequence having at least 90%, 91%, 92%, 93%, 94%, 95%, 96%, 97%, 98%, or 99% identity thereto, and SEQ ID NO: 12 or a light chain sequence having at least 90%, 91%, 92%, 93%, 94%, 95%, 96%, 97%, 98%, or 99% identity thereto;
for example, the heavy chain sequence set forth in SEQ ID NO: 11 and the light chain sequence set forth in SEQ ID NO: 12;
the heavy chain sequence set forth in SEQ ID NO: 25 and the light chain sequence set forth in SEQ ID NO: 27;
the heavy chain sequence set forth in SEQ ID NO: 25 and the light chain sequence set forth in SEQ ID NO: 28;
the heavy chain sequence set forth in SEQ ID NO: 25 and the light chain sequence set forth in SEQ ID NO: 29;
the heavy chain sequence set forth in SEQ ID NO: 25 and the light chain sequence set forth in SEQ ID NO: 30;
the heavy chain sequence set forth in SEQ ID NO: 25 and the light chain sequence set forth in SEQ ID NO: 31;
the heavy chain sequence set forth in SEQ ID NO: 26 and the light chain sequence set forth in SEQ ID NO: 27;
the heavy chain sequence set forth in SEQ ID NO: 26 and the light chain sequence set forth in SEQ ID NO: 28;
the heavy chain sequence set forth in SEQ ID NO: 26 and the light chain sequence set forth in SEQ ID NO: 29;
the heavy chain sequence set forth in SEQ ID NO: 26 and the light chain sequence set forth in SEQ ID NO: 30;
the heavy chain sequence set forth in SEQ ID NO: 26 and the light chain sequence set forth in SEQ ID NO: 31;
(b) SEQ ID NO: 13 or a heavy chain sequence having at least 90%, 91%, 92%, 93%, 94%, 95%, 96%, 97%, 98%, or 99% identity thereto, and SEQ ID NO: 14 or a light chain sequence having at least 90%, 91%, 92%, 93%, 94%, 95%, 96%, 97%, 98%, or 99% identity thereto; or
(c) SEQ ID NO: 15 or a heavy chain sequence having at least 90%, 91%, 92%, 93%, 94%, 95%, 96%, 97%, 98%, or 99% identity thereto, and SEQ ID NO: 16 or a light chain sequence having at least 90%, 91%, 92%, 93%, 94%, 95%, 96%, 97%, 98%, or 99% identity thereto; preferably, the isolated antibody or the antigen-binding fragment is a fully human antibody.

4. The isolated antibody or the antigen-binding fragment of the isolated antibody according to any one of claims 1 to 3, wherein the isolated antibody or the antigen-binding fragment of the isolated antibody is an IgG antibody, preferably a human IgG antibody, and more preferably a human IgG1 or human IgG4 antibody; the antigen-binding fragment is a Fab, a Fab', a F(ab')2, an Fv, a single-chain Fv, or a single-chain Fab.

5. The isolated antibody or the antigen-binding fragment of the isolated antibody according to any one of claims 1 to 4, having one or more of the following properties:
(a) having an ability to bind to a human Nectin-4 molecule expressed on a cell and a cynomolgus monkey Nectin-4 molecule expressed on a cell, as measured by flow cytometry;
(b) having an ability to specifically bind only to Nectin-4, with no binding to Nectin family members Nectin-1, Nectin-2, and Nectin-3, as measured by ELISA; and/or
(c) binding to a human Nectin-4 protein with a binding dissociation equilibrium constant K_{D} of less than about 10 nM, such as about 1 nM, about 10⁻¹ nM, about 10⁻² nM, or about 10⁻³ nM; and/or binding to a cynomolgus monkey Nectin-4 protein with a binding dissociation equilibrium constant K_{D} of less than about 100 nM, such as about 10 nM, about 1 nM, about 10⁻¹ nM, or about 10⁻² nM, as measured by biolayer interferometry;
wherein preferably, the isolated antibody or the antigen-binding fragment is used for preparing a multispecific antibody or an antibody-drug conjugate (ADC).

6. A multispecific antibody, comprising an isolated antibody or an antigen-binding fragment specifically binding to a Nectin-4 protein, for example, comprising the isolated antibody or the antigen-binding fragment specifically binding to the Nectin-4 protein according to any one of claims 1 to 5, wherein preferably, the multispecific antibody is a trispecific antibody or a bispecific antibody;
for example, the multispecific antibody is a bispecific antibody comprising an isolated antibody or an antigen-binding fragment specifically binding to a Nectin-4 protein and an isolated antibody or an antigen-binding fragment specifically binding to a T cell co-stimulatory receptor, such as ICOS, 4-1BB, CD28, or CD86; for example, the bispecific antibody comprises the isolated antibody or the antigen-binding fragment specifically binding to the Nectin-4 protein according to any one of claims 1 to 5 and an isolated antibody or an antigen-binding fragment specifically binding to a T cell co-stimulatory receptor, such as ICOS, 4-1BB, CD28, or CD86.

7. The multispecific antibody according to claim 6, wherein the multispecific antibody is a bispecific antibody comprising, from the amino-terminus to the carboxyl-terminus:
(a) a first moiety, which is an isolated antibody or an antigen-binding fragment specifically binding to a Nectin-4 protein, e.g., the isolated antibody or the antigen-binding fragment specifically binding to the Nectin-4 protein according to any one of claims 1 to 5, and
(b) a second moiety, which is an isolated antibody or an antigen-binding fragment specifically binding to a T cell co-stimulatory receptor 4-1BB;
wherein the antigen-binding fragments in the first moiety and the second moiety are a Fab, a Fab', a F(ab')2, an Fv, a single-chain Fv, or a single-chain Fab;
for example, the bispecific antibody comprises, from the amino-terminus to the carboxyl-terminus:
(a) a first moiety, which is the isolated antibody or the antigen-binding fragment specifically binding to the Nectin-4 protein according to any one of claims 1 to 5, and
(b) a second moiety, which is an anti-4-1BB scFv;
for example, the anti-4-1BB scFv comprises 3 CDRs in the amino acid sequence of a heavy chain variable region set forth in SEQ ID NO: 32 and 3 CDRs in the amino acid sequence of a light chain variable region set forth in SEQ ID NO: 33; or a variant with one or more CDRs each having no more than 3 amino acid modifications relative to the 6 CDR regions described above; for example, the anti-4-1BB scFv comprises, according to Kabat numbering, an HCDR1 set forth in SEQ ID NO: 63 or a variant thereof having no more than 2 amino acid modifications, an HCDR2 set forth in SEQ ID NO: 64 or a variant thereof having no more than 2 amino acid modifications, and an HCDR3 set forth in SEQ ID NO: 65 or a variant thereof having no more than 2 amino acid modifications; an LCDR1 set forth in SEQ ID NO: 66 or a variant thereof having no more than 2 amino acid modifications, an LCDR2 set forth in SEQ ID NO: 67 or a variant thereof having no more than 2 amino acid modifications, and an LCDR3 set forth in SEQ ID NO: 68 or a variant thereof having no more than 2 amino acid modifications; for example, the anti-4-1BB scFv comprises, according to Kabat numbering, an HCDR1 set forth in SEQ ID NO: 63, an HCDR2 set forth in SEQ ID NO: 64, and an HCDR3 set forth in SEQ ID NO: 65; an LCDR1 set forth in SEQ ID NO: 66, an LCDR2 set forth in SEQ ID NO: 67, and an LCDR3 set forth in SEQ ID NO: 68;
for example, the anti-4-1BB scFv comprises a heavy chain variable region set forth in SEQ ID NO: 32 or a sequence having at least 90%, 91%, 92%, 93%, 94%, 95%, 96%, 97%, 98%, or 99% identity thereto, and a light chain variable region set forth in SEQ ID NO: 33 or a sequence having at least 90%, 91%, 92%, 93%, 94%, 95%, 96%, 97%, 98%, or 99% identity thereto;
wherein the carboxyl-terminus of the heavy chain of the first moiety and the amino-terminus of the second moiety are linked via a linker or directly linked without a linker; for example, the linker is a linker peptide set forth in SEQ ID NO: 69 or SEQ ID NO: 70.

8. The multispecific antibody according to claim 7, wherein the bispecific antibody comprises two heavy chains and two light chains, wherein each heavy chain comprises, from the amino-terminus to the carboxyl-terminus, the heavy chain of the first moiety, the linker peptide, and the second moiety, and each light chain is the light chain of the first moiety; preferably, each heavy chain comprises the amino acid sequence set forth in SEQ ID NO: 35 or a sequence having at least 80%, 85%, 90%, 91%, 92%, 93%, 94%, 95%, 96%, 97%, 98%, 99%, or higher identity thereto; and each light chain comprises the amino acid sequence set forth in SEQ ID NO: 12 or a sequence having at least 80%, 85%, 90%, 91%, 92%, 93%, 94%, 95%, 96%, 97%, 98%, 99%, or higher identity thereto;
each heavy chain comprises the amino acid sequence set forth in SEQ ID NO: 36 or a sequence having at least 80%, 85%, 90%, 91%, 92%, 93%, 94%, 95%, 96%, 97%, 98%, 99%, or higher identity thereto; and each light chain comprises the amino acid sequence set forth in SEQ ID NO: 16 or a sequence having at least 80%, 85%, 90%, 91%, 92%, 93%, 94%, 95%, 96%, 97%, 98%, 99%, or higher identity thereto; or
each heavy chain comprises the amino acid sequence set forth in SEQ ID NO: 37 or a sequence having at least 80%, 85%, 90%, 91%, 92%, 93%, 94%, 95%, 96%, 97%, 98%, 99%, or higher identity thereto; and each light chain comprises the amino acid sequence set forth in SEQ ID NO: 31 or a sequence having at least 80%, 85%, 90%, 91%, 92%, 93%, 94%, 95%, 96%, 97%, 98%, 99%, or higher identity thereto.

9. The multispecific antibody according to claim 7 or 8, wherein the bispecific antibody has one or more of the following properties:
(a) binding to a human Nectin-4 protein with a binding dissociation equilibrium constant K_{D} of less than about 10 nM, such as about 1 nM, about 10⁻¹ nM, about 10⁻² nM, or about 10⁻³ nM, and binding to a human 4-1BB protein with a binding dissociation equilibrium constant K_{D} of less than about 1000 nM, such as about 100 nM, about 10 nM, about 1 nM, or about 10⁻¹ nM; or binding to a cynomolgus monkey Nectin-4 protein with a binding dissociation equilibrium constant K_{D} of less than about 100 nM, such as about 10 nM, about 1 nM, about 10⁻¹ nM, or about 10⁻² nM, and binding to a cynomolgus monkey 4-1BB protein with a binding dissociation equilibrium constant K_{D} of less than about 150 nM, such as about 15 nM, about 1.5 nM, about 0.15 nM, or about 0.015 nM, as measured by biolayer interferometry;
(b) the bispecific antibody being able to activate a human 4-1BB signaling pathway only in the presence of a Nectin-4 protein; the bispecific antibody being not able to activate the human 4-1BB signaling pathway in the absence of the Nectin-4 protein, as measured in an NF-κB-Luc/4-1BB reporter gene cell line; and/or
(c) having *in vivo* anti-tumor efficacy, and the bispecific antibody being safe to test animals.

10. An isolated nucleic acid encoding the isolated antibody or the antigen-binding fragment according to any one of claims 1 to 5 or encoding the multispecific antibody according to any one of claims 6 to 9.

11. A vector, comprising the nucleic acid according to claim 10, wherein preferably, the vector is an expression vector.

12. A host cell, comprising the nucleic acid according to claim 10 or the vector according to claim 11, wherein preferably, the host cell is prokaryotic or eukaryotic; more preferably, the host cell is selected from an *Escherichia coli* cell, a yeast cell, a mammalian cell, and other cells suitable for preparing an antibody, an antigen-binding fragment, or a multispecific antibody; and most preferably, the host cell is an HEK 293 cell or a CHO cell.

13. A method for preparing the isolated antibody or the antigen-binding fragment according to any one of claims 1 to 5 or the multispecific antibody according to any one of claims 6 to 9, comprising culturing the host cell according to claim 12 under conditions suitable for expressing a nucleic acid encoding the isolated antibody or the antigen-binding fragment according to any one of claims 1 to 5 or encoding the multispecific antibody according to any one of claims 6 to 9, and optionally recovering the isolated antibody or the antigen-binding fragment according to any one of claims 1 to 5 or the multispecific antibody according to any one of claims 6 to 9 from the host cell or a culture medium.

14. A pharmaceutical composition, comprising the isolated antibody or the antigen-binding fragment according to any one of claims 1 to 5 or the multispecific antibody according to any one of claims 6 to 9, and a pharmaceutically acceptable carrier, or
a pharmaceutical composition, comprising the isolated antibody or the antigen-binding fragment according to any one of claims 1 to 5 or the multispecific antibody according to any one of claims 6 to 9, an additional therapeutic agent selected from an oncolytic drug, a cytotoxic agent, a cytokine, and an inhibitor of other immune checkpoint molecules (e.g., PD-1), and a pharmaceutically acceptable carrier.

15. Use of the isolated antibody or the antigen-binding fragment according to any one of claims 1 to 5 or the multispecific antibody according to any one of claims 7 to 10 in preparing a medicament for diagnosing, preventing, and/or treating a disease associated with high Nectin-4 expression, wherein the disease is, for example, a cancer; for example, the cancer is a solid tumor or a hematological cancer; for example, the cancer is colon cancer, rectal cancer, colorectal cancer, esophageal cancer, skin cancer, urothelial carcinoma, ovarian cancer, pancreatic cancer, bladder cancer, non-Hodgkin's lymphoma, Hodgkin's lymphoma, acute lymphocytic leukemia, multiple myeloma, breast cancer, gastric cancer, hepatocellular carcinoma, non-small cell lung cancer, small cell lung cancer, melanoma, glioblastoma, renal cell carcinoma, or prostate cancer.
